# EUROPEAN PATENT SPECIFICATION

(11) **EP 4 578 256 B1**
(45) Date of publication and mention of the grant of the patent: **29.04.2026**
(21) Application number: 23776097.0
(22) Date of filing: 25.08.2023
(51) Int. Cl.: H10K 85/10, H10K 10/00, C09K 11/06, C08G 61/00, H10K 85/60

(54) **ORGANIC LAYER COMPRISING REGIONS HAVING DIFFERENT ELECTRONIC PROPERTIES**
ORGANISCHE SCHICHT MIT BEREICHEN MIT UNTERSCHIEDLICHEN ELEKTRONISCHEN EIGENSCHAFTEN
COUCHE ORGANIQUE COMPRENANT DES RÉGIONS AYANT DES PROPRIÉTÉS ÉLECTRONIQUES DIFFÉRENTES

(30) Priority: 25.08.2022 IT 202200017577
(43) Date of publication of application: 02.07.2025
(73) Proprietor: Fondazione Istituto Italiano di Tecnologia, 16163 Genova (IT)
(72) Inventor: CARLOTTI, Marco, 56125 PISA (IT); MATTOLI, Virgilio, 56124 PISA (IT)
(74) Representative: Biggi, Cristina
(86) International application number: PCT/IB2023/058431
(87) International publication number: WO 2024/042491

(56) References cited:
- US-A- 5 001 243
- LI JINFENG ET AL: "Enhanced stability of a rubrene analogue with a brickwork packing motif", vol. 5, no. 33, 1 January 2017 (2017-01-01), GB, pages 8376 - 8379, XP093037315, ISSN: 2050-7526, Retrieved from the Internet <URL:https://pubs.rsc.org/en/content/articlepdf/2017/tc/c7tc01790a> DOI: 10.1039/C7TC01790A
- RAMAR ALAGAR ET AL: "Advances in polymer electrode materials for alkali metals (lithium, sodium and potassium)-ion rechargeable batteries", JOURNAL OF MATERIALS SCIENCE: MATERIALS IN ELECTRONICS, vol. 31, no. 24, 13 November 2020 (2020-11-13), pages 21832 - 21855, XP037316518, ISSN: 0957-4522, DOI: 10.1007/S10854-020-04805-6
- GLÖCKLHOFER FLORIAN ET AL: "Multigram synthesis of bis[(trimethylsilyl)ethynyl]benzenes suitable for post-polymerization modification", vol. 38, no. 6, 1 January 2014 (2014-01-01), GB, pages 2229 - 2232, XP093037399, ISSN: 1144-0546, Retrieved from the Internet <URL:https://pubs.rsc.org/en/content/articlepdf/2014/nj/c4nj00011k> DOI: 10.1039/C4NJ00011K
- JU JIN UK ET AL: "Synthesis and characterization of a new ethynyl-linked alternating anthracene/fluorene copolymer for organic thin film transistor", JOURNAL OF POLYMER SCIENCE PART A: POLYMER CHEMISTRY, vol. 47, no. 6, 15 March 2009 (2009-03-15), US, pages 1609 - 1616, XP093037779, ISSN: 0887-624X, DOI: 10.1002/pola.23135

## Description

The project from which the present patent application derives received funding from the European Union Horizon 2020 research and innovation programme according to Marie Sk 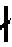 odowska-Curie grant agreement No 885881.

### FIELD OF THE INVENTION

The present invention relates to a process for the production of an organic layer comprising regions with different electronic properties. The process is based on the use of dihydroanthracene, dihydrobenzene, benzol or fluorene precursors functionalised with at least one substituted or non-substituted propargyl alcohol which provide, following a reduction or retro-Favorskii reaction, molecules and/or polymers characterised by a high HOMO or low LUMO.

### BACKGROUND OF THE INVENTION

Research on organic electronics is one of the most important examples where a fundamental understanding of physical phenomena on a nanometric scale and the preparation of real functional devices are combined. Conjugated polymers and small organic molecules can offer interesting and customisable electronic properties and, in combination with the processability and mechanical properties of organic compounds, they are used in various cutting-edge technologies as flexible electronic devices, economical and single-use components and sensors, photovoltaic elements, electrodes for batteries and wearable accessories.

However, when one speaks of different technologies, the performances of organic materials are not on a par with the possibilities offered by their inorganic counterparts.

A clear example of their limits is given by the current lack of solutions for implementing regions with complementary electronic properties (i.e. n- and p-doped regions) on the same semiconductor layer, as occurs in the case of CMOS (Complementary Metal-Oxide Semiconductor) technology for silicon. This is because every material is defined by a unique set of chemical and electronic properties.

In the manufacture of real devices, this precludes the realisation of some designs and increases the number of manufacturing steps necessary (which, moreover, sets a lower limit on scalability).

It is well known that small bandgap π conjugated polymers are an important class of materials with significant potential for their broad application in organic electronics. Bandgap is in fact one of the most important properties of π conjugated polymers. This characteristic refers qualitatively to the energy gap between the HOMO and LUMO levels. When the bandgap is lowered, the frontier orbitals of a material can become energetically accessible to charge transfer and other phenomena that lead to unique devices.

Various attempts have been made to obtain increasingly high-performing products for applications in optoelectronic devices, such as light-emitting diodes, organic field-effect transistors, organic photovoltaics, and many others. Moreover, the preparation of π conjugated polymers with a zero or very small intrinsic bandgap would lead to metallic behaviour, thus providing an intrinsic electric conductivity without doping.

An article by Tsubasa Mikie et al., "Small-bandgap quinoid-based π-conjugated polymers", Journal of Materials Chemistry C, 2020, illustrates the progress in small-bandgap quinoid-based π-conjugated polymers, focusing on a variety of electron-deficient quinoid building units; the article highlights the properties of each quinoid unit and the polymers, as well as their structural features and device performances.

However, the document does not suggest a way to incorporate different organic products, derived from the same precursor, into a single engineered layer, which resembles an inorganic semiconductor layer having, respectively, n- and p-doped regions.

An article by Yasunori Takeda et al., "Fabrication of ultra-thin printed organic TFT CMOS logic circuits optimized for low-voltage wearable sensor applications", *Scientific Reports* 2016, describes complementary metal-oxide-semiconductor (CMOS) logic circuits in which p-type and n-type thin film organic transistor devices are used, but according to a multilayer structure, and which show excellent electrical characteristics. The p-type semiconductor consists of an ink comprising a mixture of diF-TES-ADT and PS - a known high-performance organic semiconductor - whereas the n-type semiconductor consists of a solution having benzobis(thiadiazole) as its basic chemical structure, but which is soluble in many common organic solvents.

An article by Philip Hodge et al., "Polymers containing in-chain quinone moieties: synthesis and properties", *Polym Int* 2009, focuses on the synthesis and properties of polymers that contain in-chain quinone units, but clearly shows that the precursors produce only one type of polymeric material, which thus does not have adjustable electronic properties.

An article by Mitsuharu Suzuki et al., "A photochemical layer-by-layer solution process for preparing organic semiconducting thin films having the right material at the right place", Royal Society of Chemistry, 2018, discloses a photoreaction-based layer-by-layer solution process. In particular, the article focuses attention on the synthesis and photoreactivity of two DK-type (α-diketone) derivatives of small-molecule organic semiconductors PhBADT and AtD, which are specifically chosen/designed for the p- and i- layers in p-i-n-type OPVs. The versatility of the technique described enabled the controlled deposition of ternary photovoltaic layers containing two independently optimised different p-type materials.

In this case as well, the precursors produce only one type of material and there are no suggestions as to how to produce a single organic layer with regions with different and complementary electronic properties. Furthermore, LI JINFENG et al. in "Enhanced stability of a rubrene analogue with a brickwork packing motif", vol. 5, no. 33 (2017-01-01), pages 8376-8379, is concerned with creating a material by rearomatization reaction for OFET devices. JU JIN UK et al. in "Synthesis and characterization of a new ethynyl-linked alternating anthracene/ fluorene copolymer for organic thin film transistor", JOURNAL OF POLYMER SCIENCE PART A: POLYMER CHEMISTRY, vol. 47, no. 6, (2009-03-15), pages 1609-1616, synthesize a conjugated polymer (PDADF), which is composed of ethynyl-linked alternating an-thracene/fluorene units for OFETs.

Therefore, in this sector there remains a need to provide a method for the production of a single organic layer having regions with different and complementary electronic properties.

### SUMMARY OF THE INVENTION

The present invention relates to a process for the production of an organic layer comprising regions with different electronic properties. The process is based on the use of dihydroanthracene, dihydrobenzene, benzol or fluorene precursors functionalised with at least one substituted or non-substituted propargyl alcohol. By subjecting these precursors to a reduction/re-aromatisation reaction and a retro-Favorskii reaction one obtains small organic molecules having different electronic properties, due to the specific chemical composition generated by the various chemical reactions in which the precursors are involved. Therefore, starting from the same precursor and subjecting it alternatively to the two reactions specified, one obtains products with different electronic properties, i.e. molecules characterised by high energy levels of the highest occupied molecular orbital (HOMO), which have at least one aromatic ring substituted with at least one ethynyl group, and molecules characterised by lower energy levels of the lowest unoccupied molecular orbital (LUMO), which have at least one carbonyl functionality, preferably at least one quinone ring or at least one phenyl with at least one carbonyl functionality.

The reactions are carried out in such a way that the final products are incorporated into a single organic layer.

In one embodiment, the dihydroanthracene, dihydrobenzene, benzol or fluorene precursors are subjected to a homo-polymerisation or co-polymerisation reaction with a different monomer, before undergoing the aforesaid reduction/re-aromatisation and retro-Favorskii reactions. Alternatively, the homo-polymerisation or co-polymerisation reaction can be carried out after the precursors have been subjected to the reduction or retro-Favorskii reaction. In both cases, polymers/oligomers with different electronic properties are generated, that is, polymers characterised by higher HOMO energy levels, comprising at least one ring substituted with at least one ethynyl group, and polymers characterised by lower LUMO energy levels, comprising at least one carbonyl functionality, preferably at least one quinone ring or at least one phenyl ring with at least one carbonyl functionality.

With this embodiment as well, a single organic layer comprising regions with different electronic properties is generated.

The subject matter of the invention relates to a single organic layer comprising a plurality of regions with different and complementary electronic properties, such regions comprising organic molecules and/or organic polymers characterised by high energy levels of the highest occupied molecular orbital (HOMO) and comprising organic molecules and/or organic polymers characterised by lower energy levels of the lowest unoccupied molecular orbital (LUMO).

The single organic layer is used for the production of electronic devices, such as, for example, organic transistors in which the layer carries out both a p-type and an n-type transport according to the electronic properties of the various regions making up the layer. Transistors in which the single organic layer is used are organic field-effect transistors (OFET) and organic electrochemical transistors (OECT).

Other electronic devices where the organic layer of the invention can be used are for example light-emitting diodes and organic photovoltaic panels.

### BRIEF DESCRIPTION OF THE FIGURES

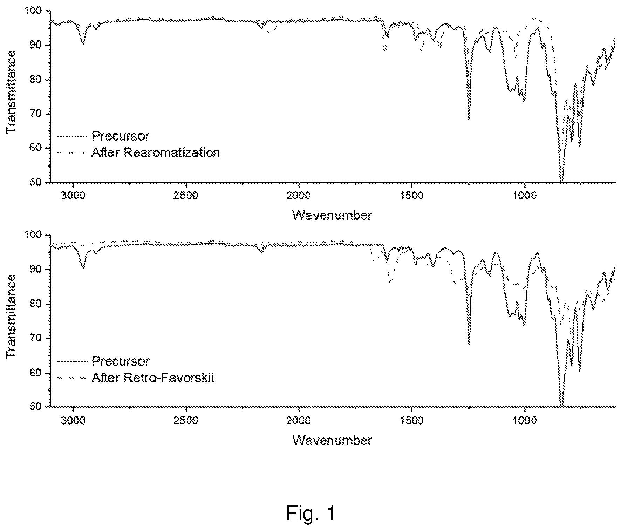

Figure 1. ATR-IR spectra of the precursor polymer of example P before and after reduction/aromatisation (example Q, spectrum at the top) and before and after retro-Favorskii (example R, spectrum at the bottom).

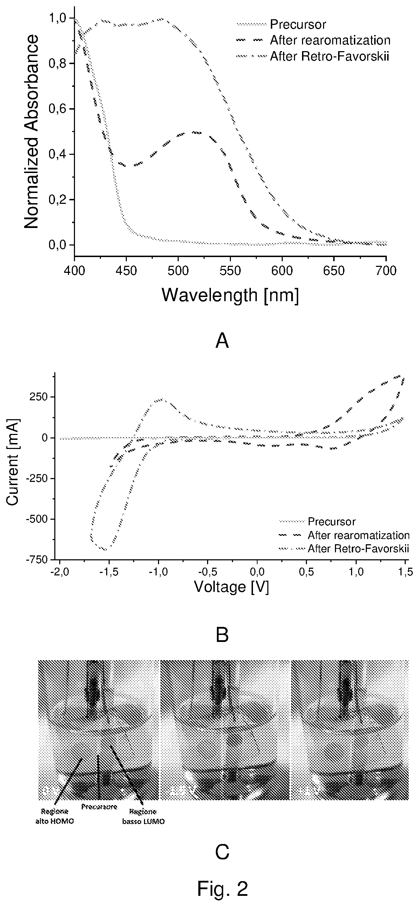

Figure 2. A - Comparison of the UV-Vis spectra of thin films of the precursor polymer of example P (light grey), after reduction/aromatisation (example S, dark grey) and retro-Favorskii (example T, grey); B - Comparison of the CV spectra of conductive indium-tin oxide thin films of the precursor polymer (example P, light grey), after reduction/re-aromatisation (example S, dark grey) and retro-Favorskii (example T, grey) in acetonitrile using 0.1. M tetrabutylammonium hexafluorophosphate as the electrolyte and a scanning rate of 0.3 mV/s; C - Electrochromic behaviour in a solution of 0.1 M tetrabutylammonium hexafluorophosphate in acetonitrile of films obtained by spin coating of the precursor of example C with regions treated in different ways (reference: Ag/Ag+ electrode, counter electrode: Pt wire). In particular, in the circular region on the left is a film treated under reduction-aromatisation conditions to obtain the conjugated polymer with a high HOMO (example U), while in the oval region on the right is a film treated under retro-Fovrskii conditions to obtain the conjugated polymer with a low LUMO (example U). Each panel shows the voltage, with respect to Ag/Ag+, at which the picture was taken.

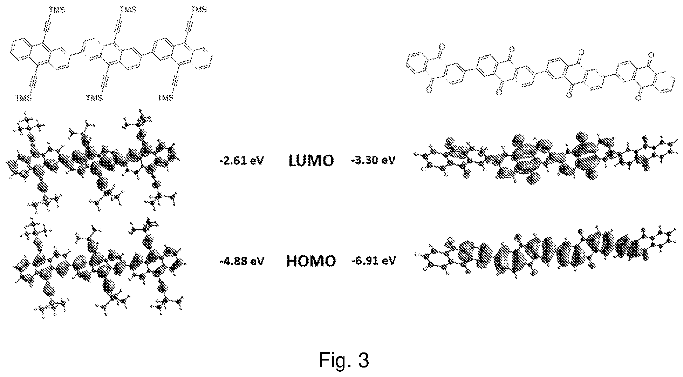

Figure 3 shows the chemical structure (at the top), and the energies and distributions of LUMO (in the middle) and HOMO (at the bottom) in two examples of model oligomers comprising the units 9,10-di(ethynyl-2-trimethylsilyl)-anthracene and 9,10-anthraquinone. For the trimer of the respective precursor (which contains an ethynyl and a hydroxyl), the LUMO and HOMO are calculated at -1.67 and -6.47 eV, respectively.

### DETAILED DESCRIPTION OF THE INVENTION

For the purposes of the present invention, "single organic layer", "organic film" and "film" are used interchangeably and refer to a layer/film that incorporates different and complementary electronic properties thanks to the presence of a plurality of regions with different electronic properties.

The definitions: "high energy levels of the highest occupied molecular orbital (HOMO)", "high-energy HOMO", "rich electron density", and "p behaviour" are considered interchangeable.

The definitions: "lower energy levels of the lowest unoccupied molecular orbital (LUMO)", "low-energy LUMO", "poor electron density", and "n behaviour" are considerate interchangeable.

The invention relates to a process for the production of an organic layer/film comprising a plurality of regions with different and complementary electronic properties, in particular one or more regions characterised by relatively high energy levels of the highest occupied molecular orbital (HOMO), and which comprise molecules and/or polymers comprising at least one aromatic ring substituted with at least one ethynyl group, and one or more regions characterised by relatively low energy levels of the lowest unoccupied molecular orbital (LUMO), and which comprise molecules and/or polymers comprising at least one carbonyl functionality, preferably at least one quinone ring or at least one phenyl with at least one carbonyl functionality.

The molecules with electron donor characteristics (high HOMO) preferably comprise at least two aromatic rings, preferably condensed to yield a naphthalene unit, substituted with at least one ethynyl group. In one embodiment, the molecules comprise three aromatic rings preferably condensed to yield an anthracene unit substituted with at least one ethynyl group. In one embodiment, the polymers comprise a plurality of repetitive naphthalene and/or anthracene units, each substituted with at least one ethynyl unit.

The process is based on the use of at least one precursor having a general formula selected from the following formulas (I), (II), (IIa), (III) and (IV). 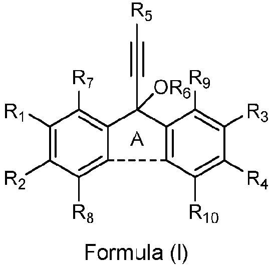 in which
R1, R2, R3, R4, R5, R7, R8, R9 and R10 are independently selected from: H, a halogen selected from Br, I, Cl, and F, C1-C8 alkyl, vinyl, alkynyl, aryl, heteroaryl, benzyl, N, B, S, O, Sn, and tri-alkyl-silane;
R6 is selected from: H, C1-C8 alkyl, vinyl, alkynyl, aryl, benzyl, and tri-alkyl-silane;
A indicates a 5-carbon atom ring or a 6-carbon atom ring optionally substituted with a propargyl alcohol group, in which the alcohol of the propargyl alcohol group is optionally substituted, or the A ring is not present.

In one embodiment, the halogen is selected from Br and Cl.

In one embodiment, the alkyl group is a C1-C6 alkyl, or a C1-C4 alkyl, or it is methyl, ethyl, propyl, isopropyl or butyl.

In one embodiment, the tri-alkyl-silane is a tri-methyl-silane, a tri-ethyl-silane, a tri-isopropyl-silane, or a di-methyl-butyl-silane.

In one embodiment, R2 and R3 are H or a halogen selected from Br, I, and Cl, they are preferably both Br, R1, R4 and R7-R10 are H, and R5 and R6 are a tri-alkyl-silane, preferably a tri-methyl-silane and/or a tri-isopropyl-silane, A is a saturated 5-carbon atom ring or a saturated 6-carbon atom ring optionally substituted with a propargyl alcohol group, in which the alcohol of the propargyl alcohol group is optionally substituted, or the ring A is not present. In one embodiment, A is present as a saturated 5-carbon atom ring or A is not present.

Examples of precursors of formula (I) in which A is a 5-atom ring or is absent are the following:

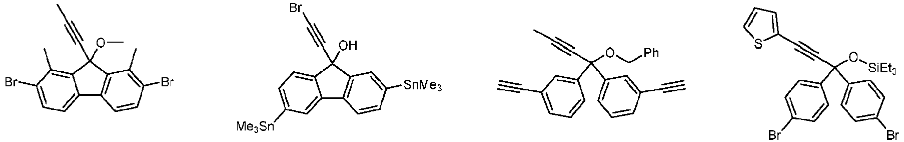

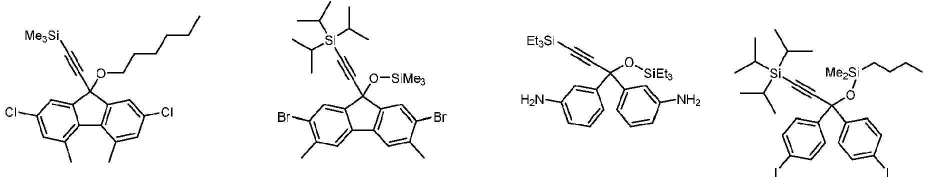

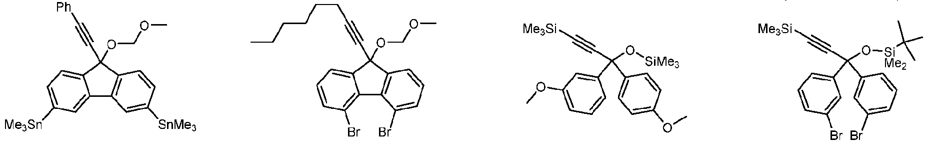

In one embodiment, A is a saturated 6-carbon atom ring substituted with a propargyl alcohol group substituted or not substituted according to the formula (la): 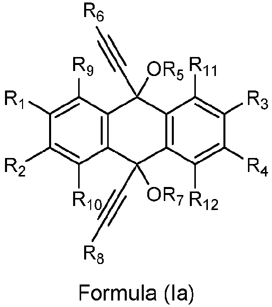 in which:
R1, R2, R3, R4, R6, R8, R9, R10, R11 and R12 are independently selected from: H, a halogen selected from Br, I, Cl, and F, C1-C8 alkyl, vinyl, alkynyl, aryl, heteroaryl, benzyl, N, B, S, O, Sn, and tri-alkyl-silane;
R5 and R7 are independently selected from: H, C1-C8 alkyl, vinyl, alkynyl, aryl, benzyl, and tri-alkyl-silane.

In one embodiment, the halogen is selected from Br and Cl.

In one embodiment, the alkyl group is a C1-C6 alkyl, or a C1-C4 alkyl, or it is: methyl, ethyl, propyl or butyl.

In one embodiment, the tri-alkyl-silane is a tri-methyl-silane, a tri-ethyl-silane, or a tri-isopropyl-silane.

In one embodiment, R2 and R3 are a halogen selected from Br, I, and Cl, they are preferably both Br, R1, R4 and R9-R12 are H, and R5, R6, R7 and R8 are a tri-alkyl-silane, preferably a tri-methyl-silane and/or a tri-isopropyl-silane.

In one embodiment, R1, R2, R3, R4, R6, R8 and R9-R12 are H, and R5 and R7 are a tri-alkyl-silane, preferably selected from tri-methyl-silane and/or tri-isopropyl-silane.

Examples of precursors of formula (la) are shown below: 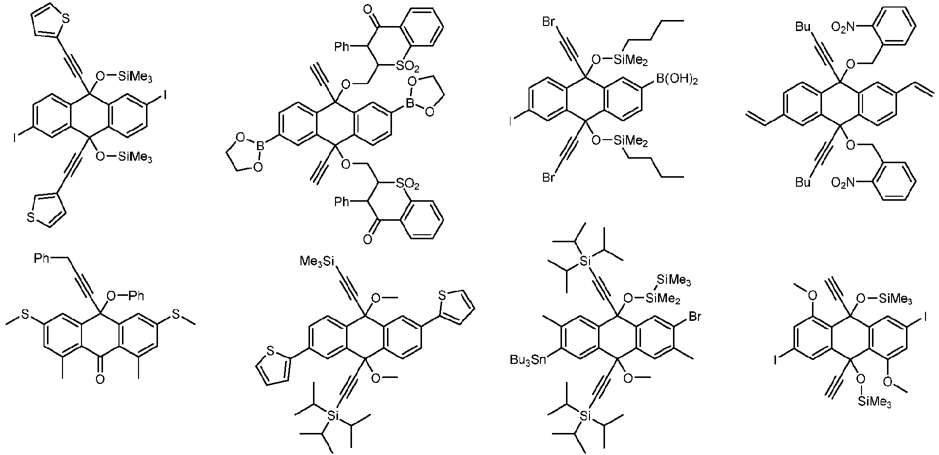 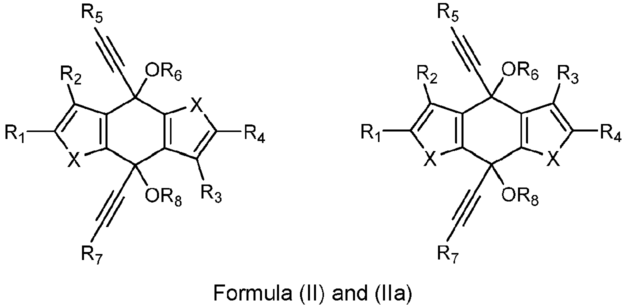 in which
R1, R2, R3, R4, R5 and R7 are independently selected from: H, a halogen selected from Br, I, Cl, and F, C1-C8 alkyl, vinyl, alkynyl, aryl, heteroaryl, benzyl, N, B, S, O, Sn, and tri-alkyl-silane;
R6 and R8 are independently selected from: H, C1-C8 alkyl, vinyl, alkynyl, aryl, benzyl, and tri-alkyl-silane;
X is selected from S, Se, N, O, and methylene.

In one embodiment, the halogen is selected from Br and Cl.

In one embodiment, the alkyl group is a C1-C6 alkyl, or a C1-C4 alkyl, or is selected from: methyl, ethyl, propyl, and butyl.

In one embodiment, the tri-alkyl-silane is a tri-methyl-silane, a tri-ethyl-silane, or a tri-isopropyl-silane.

In one embodiment, R1 and R4 are H or a halogen selected from Br and Cl, they are preferably both Br, and R5, R6, R7 and R8 are a tri-alkyl-silane, preferably a tri-methyl-silane and/or a tri-isopropyl-silane.

Examples of precursors of formula (II) and (IIa) are shown below: 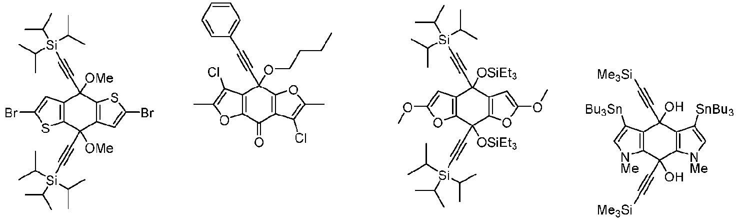 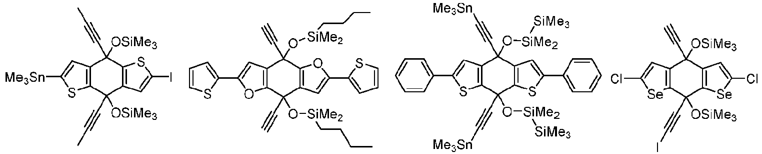 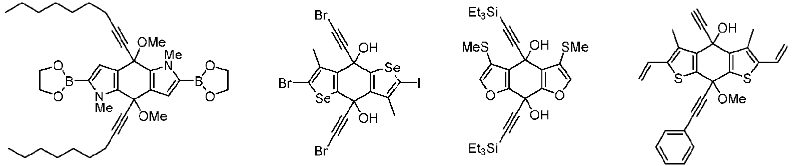 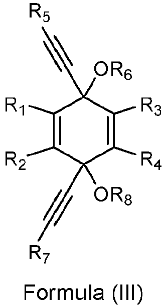 in which
R1, R2, R3, R4, R5 and R7 are independently selected from: H, a halogen selected from Br, I, Cl, and F, C1-C8 alkyl, vinyl, alkynyl, aryl, heteroaryl, benzyl, N, B, S, O, Sn, and tri-alkyl-silane;
R6 and R8 are independently selected from: H, C1-C8 alkyl, vinyl, alkynyl, aryl, benzyl, and tri-alkyl-silane.

In one embodiment, the halogen is selected from Br and Cl.

In one embodiment, the alkyl group is a C1-C6 alkyl, or a C1-C4 alkyl, or is selected from: methyl, ethyl, propyl, and butyl.

In one embodiment, the tri-alkyl-silane is a tri-methyl-silane, a tri-ethyl-silane, or a tri-isopropyl-silane.

In one embodiment, R1, R2, R3 and R4 are independently selected from H and a halogen selected from Br and Cl and R5, R6, R7 and R8 are independently selected from: tri-alkyl-silane, preferably selected from tri-methyl-silane and/or tri-isopropyl-silane.

In one embodiment, the precursor of formula (III) is selected from: 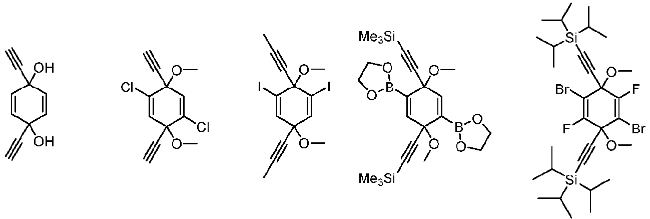 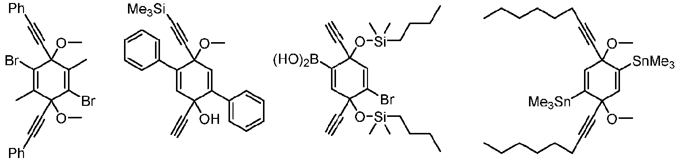 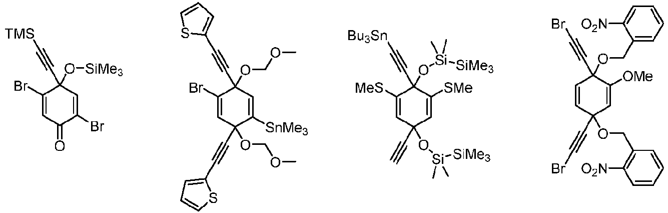 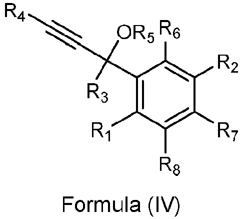 in which
R1, R2, R4, R6, R7 and R8 are independently selected from: H, a halogen selected from Br, I, Cl, and F, C1-C8 alkyl, vinyl, alkynyl, aryl, heteroaryl, benzyl, N, B, S, O, Sn, and tri-alkyl-silane;
R3 is selected from: H, C1-C8 alkyl, vinyl, alkynyl, aryl, benzyl, and N;
R5 is selected from: H, C1-C8 alkyl, vinyl, alkynyl, aryl, benzyl, and tri-alkyl-silane.

In one embodiment, the halogen is selected from Br and Cl.

In one embodiment, the alkyl group is a C1-C6 alkyl, or a C1-C4 alkyl, or is selected from: methyl, ethyl, propyl, or butyl.

In one embodiment, the tri-alkyl-silane is a tri-methyl-silane, a tri-ethyl-silane, or a tri-isopropyl-silane.

In one embodiment, R1, R2 and R6-R8 are independently selected from H and a halogen selected from Br and Cl and R3, R4 and R5 are independently selected from: tri-alkyl-silane, preferably selected from tri-methyl-silane and/or tri-isopropyl-silane, alkyl, preferably methyl and/or butyl. Examples of precursors of formula (IV) are shown below:

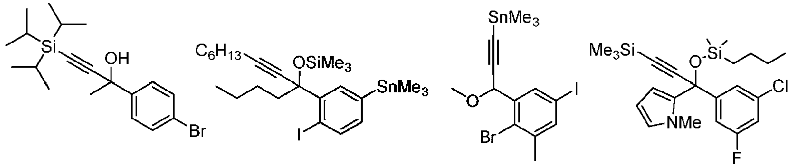

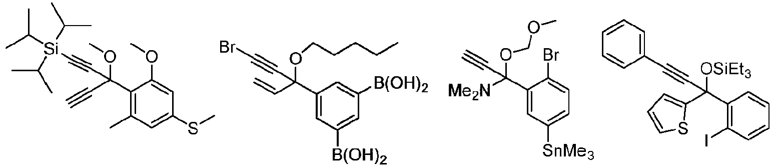

The precursors of formula (I), (la), (II), (Ila), (III) and (IV) can be prepared with methods of synthesis known in the art and described in the examples.

According to the process of the invention, the precursors of the general formula (I), (Ia), (II), (IIa) (III), and (IV), individually or in combination, are subjected to a reduction/re-aromatisation reaction and retro-Favorskii reaction in order to obtain, in the former case, molecules characterised by particularly high energy levels of the highest occupied molecular orbital (HOMO) and, in the latter case, to obtain molecules characterised by particularly low energy levels of the lowest unoccupied molecular orbital (LUMO). Therefore, using the same precursor it is possible to obtain molecules with different electronic properties depending on the reaction to which the precursor is subjected.

The reduction/re-aromatisation reaction is carried out by treating the precursor or a mixture of precursors according to the formulas (Ia), (II), (lla) and (III) with a reducing/re-aromatising agent, preferably selected from: SnCl₂/H⁺, 2- nitrobenzenesulfonylhydrazide and PCl₃.

The reaction scheme is shown in Fig. 1 below:

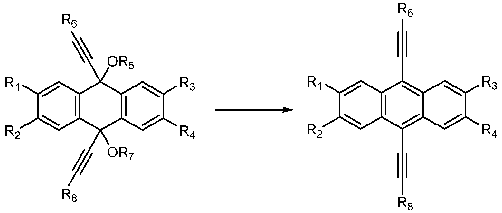

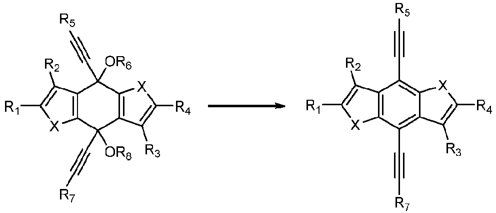

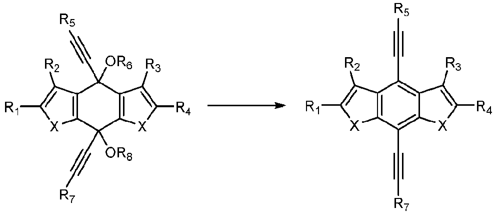

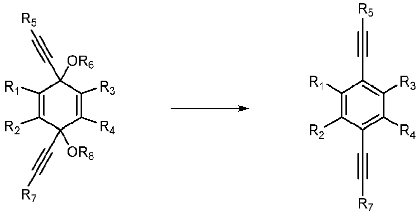

Fig. 1 - scheme of reduction/re-aromatisation reaction starting from precursors of formula (la), (II), (lla) and (III).

The reduction/re-aromatisation reaction leads to the formation of products characterised by at least one aromatic ring substituted with at least one ethynyl group, a characteristic that imparts electronic properties of electron donors identified by a high-energy HOMO. When the precursor has formula (la), the reaction contains an anthracene unit (3 condensed aromatic rings) substituted with at least two ethynyl units.

The precursor of formula (I) with A equal to a 5-carbon atom ring and the precursor of formula (IV) do not react under reduction/re-aromatisation conditions and can only be used in the retro-Favorskii reaction.

The retro-Favorskii reaction is carried out by treating the precursor or a mixture of precursors of formula (I)-(IV) under strong basic conditions (for example using KOH, NaOH and/or alkoxides), or with fluoride (for example tetrabutylammonium fluoride, KF and/or AgF).

The scheme of the retro-Favorskii reaction is shown in Fig. 2

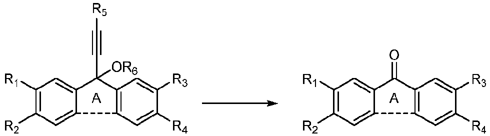

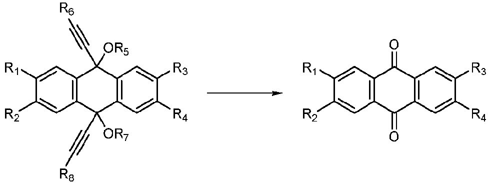

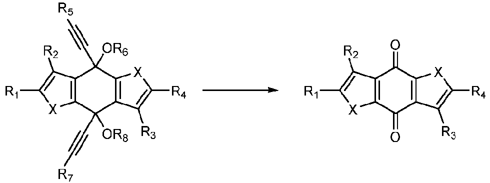

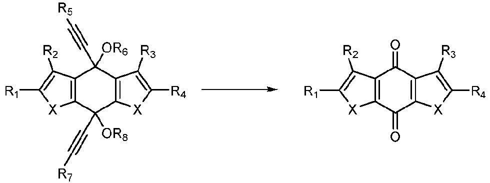

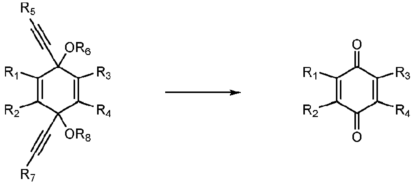

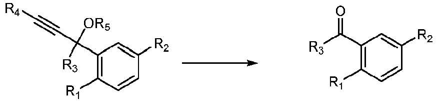

Fig. 2 - Scheme of retro-Favorskii reaction starting from precursors of formula (I), (Ia), (II), (Ila), (III) and (IV).

The retro-Favorskii reaction leads to the formation of at least one carbonyl group in the case of the precursors of formula (I) when A is a 5-carbon atom ring or is not present and in the case of the precursor of formula (IV). In the case of the precursors of formula (Ia), (II), (Ila) and (III) there is the formation of at least one quinone. The presence of at least one carbonyl functionality in the products obtained from the reaction imparts electronic properties of the electron attractor type identified by a low-energy LUMO.

In one embodiment, the precursors of formula (I), (la), (II), (IIa), (III) and (IV) are subjected to a polymerisation reaction before being subjected to the reduction/re-aromatisation reaction and retro-Favorskii reaction. The polymerisation can be a homo-polymerisation or a co-polymerisation. In the latter case, the co-monomer is selected from: ethene, ethine, benzene, acenes, thiophene, 2,2'-bithiophene, bithiazole, fluorene, thieno[3,2-b]thiophene, dithieno[3,2-b:2',3'-d]thiophene, 1,4-dione-pyrrolo[3,4-c]pyrrole and 1,3,6,8(2H,7H)-tetraone-2,7-dialkylbenzo[lmn][3,8]phenanthroline. The polymerisation conditions are those known in the art, for example based on the Stille reaction, Suzuki reaction, Kumada reaction, Heck reaction, Pd-catalysed coupling reactions and Ni-catalysed coupling reactions.

The molecular weight of the polymers obtainable from the polymerisation reaction, when carried out before the reduction and retro-Favorskii reactions, is between 2000 Da and 80000 Da, preferably between 20000 and 60000 Da (high molecular weight polymers).

In the case of the precursors of formula (I) and (la), if at least one among R1, R2, R3 and R4 is substituted with halogen, organic tin or boronic esters, the homo-polymerisation or co-polymerisation takes place on the substituted group. If R6 and/or R8 (in the case of the formula la) are not substituted or equal to Br or I, the polymerisation takes place on the R6 and/or R8 position provided that one uses, as a coupling agent, Cu(I), a base and optionally an oxidant such as oxygen or iodine (Glaser reaction, Cadiot-Chodkiewicz reaction).

Similarly, in the case of a precursor of formula (II), (Ila), (III) or (IV) the homo-polymerisation or the co-polymerisation can take place on the R groups that substitute the rings in the case where such groups are substituted with at least one halogen, organic tin or boronic esters or on the R groups of the alkynyl units in the case where the substituents are H, Br or I and one uses, as a coupling agent, Cu(I), a base and optionally an oxidant such as oxygen or iodine (Glaser reaction, Cadiot-Chodkiewicz reaction).

Once a homopolymer or copolymer has been formed starting from one or more of the precursors of formula (I), (Ia), (II), (IIa), (III) and (IV) as indicated above, one proceeds with the reduction/re-aromatisation reaction or retro-Favorskii reaction in order to obtain homopolymers or copolymers which, in the former case, are characterised by relatively high energy levels of the highest occupied molecular orbital (HOMO) and, in the latter case, by relatively low energy levels of the lowest unoccupied molecular orbital (LUMO).

Examples of co-polymers obtainable according to the embodiment whereby the polymerisation takes place before the reduction and retro-Favorskii reactions are shown here below:

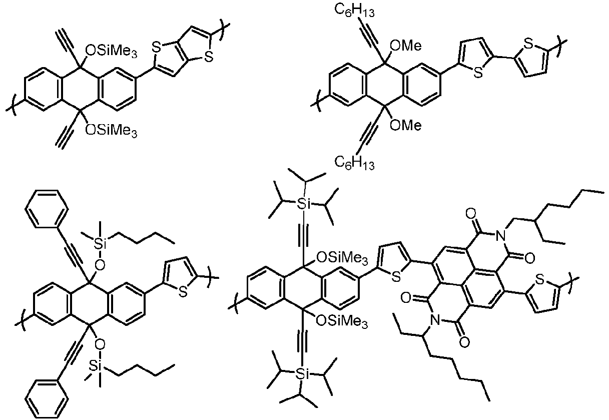

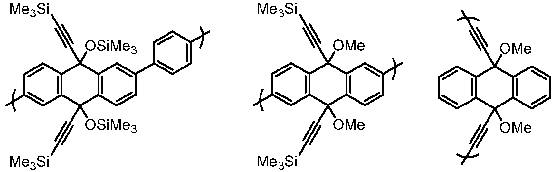

In an alternative embodiment, the homo-polymerisation or co-polymerisation reaction can be carried out after having subjected the precursors of formula (I), (la), (II), (IIa), (III) or (IV) to the reduction/re-aromatisation reaction or to the retro-Favorskii reaction.

The homopolymers or copolymers obtained with the polymerisation reaction carried out after the reduction/re-aromatisation reaction or the retro-Favorskii reaction are low molecular weight polymers, preferably between 1000 and 2000 Da (low molecular weight polymers or oligomers). These polymers have different electronic characteristics depending on the reactions applied and are used, as in the case of the products obtained by subjecting the precursors of formula (I), (Ia), (II), (IIa) (III) and (IV) to the reduction-re-aromatisation or retro-Favorskii reaction, to create a plurality of regions with different electronic characteristics within a single organic layer or film.

In one embodiment the single organic layer or film can be formed by subjecting the precursors, optionally polymerised, to the reduction or retro-Favorskii reaction to yield molecules or polymers characterised by a high HOMO and molecules or polymers characterised by a low LUMO. Once formed, the molecules or polymers characterised by a high HOMO and molecules or polymers characterised by a low LUMO are used to form a plurality of regions with a different electronic affinity within a single organic layer. The formation of the film can take place, for example, with drop casting, spin coating, screen-printing, flexography, and offset printing techniques.

Alternatively, a single organic layer or film comprising a precursor of formula (I), (Ia), (II), (IIa), (III) or (IV) or a polymer obtained by subjecting the precursors to a homo-polymerisation or co-polymerisation reaction, can be obtained, for example by drop casting. The film is subsequently stamped in different regions with stamps impregnated, respectively, with a solution based on a reducing/re-aromatising agent preferably selected from: SnCl₂/H⁺, 2-nitrobenzenesulfonylhydrazide and PCl₃, or a solution comprising a strong base or a fluoride (retro-Favorskii).

Printing is carried out in a temperature range of between 70°C and 100°C, for a time between 30 seconds and 5 minutes, preferably between 30 seconds and 2 minutes.

In this manner, one obtains a single organic film or layer comprising a plurality of regions ad high HOMO and a low LUMO having different and complementary electronic properties.

The subject matter of the invention relates to a single organic layer or film comprising a plurality of regions having different and complementary electronic properties, in which the plurality of regions comprises one or more regions with electrical and electronic properties deriving from a high HOMO and one or more regions with electrical and electronic properties deriving from a low LUMO. The one or more regions with electrical and electronic properties deriving from a high HOMO comprise molecules and/or polymers obtained from the reduction/re-aromatisation reaction of a precursor of formula (Ia), (II), (IIa) or (III), optionally polymerised before or after the reduction reaction. The one or more regions with electrical and electronic properties deriving from a low LUMO comprise molecules and/or polymers obtained from the retro-Favorskii reaction of a precursor of formula (I), (Ia), (II), (IIa), (III) or (IV), optionally polymerised before or after the retro-Favorskii reaction.

The film has a thickness of between 10 nm and 0.1 mm.

The film has application in the preparation of electronic devices, in particular transistors, preferably organic field-effect transistors (OFET) and organic electrochemical transistors (OECT).

In transistors the film performs a double n- and p-transport function.

The film also has application in the preparation of photovoltaic panels as a thin organic layer or in the formation of light-emitting diodes or in the preparation of electrochromic layers.

The polymers characterised by a low-energy LUMO obtained from the reduction reaction of the precursor of formula (I), (Ia), (II), (IIa), (III) and (IV), have a high molecular weight and a high amount of anthraquinone units, which makes them particularly suitable also as materials for battery electrodes thanks to their oxidation-reduction (redox) properties, which enable a reversible transition between the quinone and hydroquinone forms.

The high molecular weight of the polymers, which is preferably between 2000 Da and 80000 Da, makes it possible to obtain superior performances as battery electrodes compared to the materials known in the art that have lower molecular weights, since the charge storage properties are enhanced by the high number of anthraquinone units and the high molecular weight renders the polymer effectively insoluble and prevents its detachment from the electrode, thus increasing the battery life.

The redox transitions are accompanied by a notable change in colour; therefore, these polymers can also be used as electrochromic materials.

Moreover, the presence of at least one carbonyl group represents a starting point for further functionalisation (for example, imidation, Knoevenagel reaction, aldol addition, reduction, nucleophilic attack) and/or for the preparation of polyions. This possibility of further modification enables greater control over the chemical and electronic properties of the material.

As regards the polymers characterised by a high-energy HOMO obtained by reduction/re-aromatisation of the precursors (la), (II), (IIa) or (III), the presence of an extended conjugation can serve as a basis for application in organic acceptors for polymeric photovoltaic cells and for the preparation of polymeric conductors following doping. In this case as well, the redox transitions are accompanied by a notable change in colour; therefore, these polymers can also be used as electrochromic materials.

### EXAMPLES

### Example 1 - General synthesis procedures

**Step 1. Synthesis of the precursors.** The precursors functionalised with propargyl alcohol are prepared by nucleophilic attack of an acetylide on carbonyls of a sublayer appropriate for forming the propargyl alcohol. 1-3 equivalents of acetylide per carbonyl are necessary to complete the reaction, which is carried out in tetrahydrofuran at a temperature of between -21°C and 70°C and for a period of time that can range from 5 minutes to 5 hours. It is possible to use solvent-free reactions by sonicating a mixture comprising an alkyne, a strong base (for example, potassium tert-butoxide) and the desired sublayer. After the reaction, the oxygen of the alcohol group can be further functionalised with a suitable electrophile, such as methyl iodide, primary alkyl halides, chlorosilanes or masked carbocations.

**Step 2. Synthesis of the precursor polymer.** The polymerisable groups can be inserted on the precursor before or after step 1, depending on their tolerance to the above-described reaction conditions. For the synthesis, one can use standard polymerisation procedures for the preparation of conjugated materials. These reactions include, but are not limited to, palladium-catalysed couplings of halogenated compounds with organic tin, organic boron derivatives, carbon species sp2 or sp3 or nickel-catalysed reactions. Furthermore, it is also possible to use the aforesaid precursors for the preparation of non-conjugated polymers containing optoelectronically active groups.

**Step 3. Transformation into final materials.** The polymers of the precursors can be transformed into different final materials characterised by a high HOMO or a low LUMO. These transformations can be carried out on precursors in a solution, in the solid phase or in thin films. In the case of the latter, the reactions can be carried out locally and orthogonally, thus allowing the possibility of forming different phases on the same sublayer. This is achieved by drop casting, direct printing, screen-printing or exposure to heat or light.

***High HOMO materials:*** materials rich in electrons (high-energy HOMO) can be obtained from the precursor through a reduction/re-aromatisation reaction (on monomers that can undergo this transformation). The reaction requires the use of tin(II) chloride solutions (1-20 % by weight) in methanol containing 0.5-10% hydrochloric acid or sulphuric acid or in acetic acid containing 0-50 %vol. of methanol, or a solution of 2-nitrobenzenesulfonylhydrazide in methanol, nitrobenzene, acetone or methyl isobutyl ketone. The reactions are carried out at 20-100 °C and take from just a few seconds to 15 minutes. The transformation is usually accompanied by a change both in colour and in fluorescence (if present in the precursor) of the material towards the red end of the visible spectrum.

***Low LUMO materials:*** materials with a low electron content (low-energy LUMO) can be obtained from the precursors through a retro-Favorskii reaction. This is carried out using at least 1 equivalent of KOH in toluene or alcoholic solvent for every residue of propargyl alcohol at 80-120°C for at least 10 minutes, or at least 1 equivalent of tetrabutylammonium fluoride in aprotic solvents such as tetrahydrofuran or ketone solvents. The transformation is usually accompanied by a change both in colour and in fluorescence (if present in the precursor) of the material towards the red end of the visible spectrum.

### Example 2 - Specific synthesis of precursors

### Example A - Synthesis of the precursor ((2,6-dibromo-9,10-bis((butyldimethylsilyl)oxy)-9,10-dihydroanthracene-9,10-diyl)bis(ethylene-2,1-diyl))bis(triisopropylsilane):

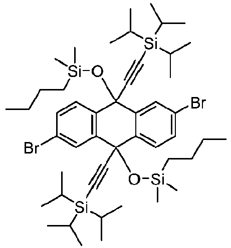

In a dry 100 mL round-bottom flask under nitrogen, 0.8 mL of triisopropylsilylacetylene were dissolved in 20 mL of dry THF and the solution was placed in an ice bath. 2.3 mL of methyllithium 1.6 M in diethyl ether were added dropwise and the reaction was left for 20 minutes. 0.5 g of 2,6-dibromoanthraquinone were added and the bath was removed. After 30 minutes, 1 mL of dimethylbutylchlorosilane was added. After 2 hours, the solution was extracted with a saturated aqueous solution of ammonium chloride and dried with magnesium sulphate. The solvent was removed under vacuum and the residue was recrystallised twice from ethanol to obtain 546 mg of product as white needles (yield: 41%).

### Example B - Synthesis of the precursor 9,10-dimethoxy-9,10-bis((trimethylsilyl)ethynyl)-9,10-dihydroanthracene:

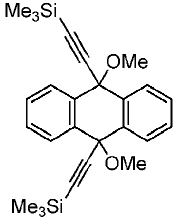

In a dry 100 mL round-bottom flask under nitrogen, 3.2 mL of trimethylsilylacetylene were dissolved in 20 mL of dry THF and the solution was placed in an ice bath. 8.8 mL of n-butyllithium 2.5 M in hexane were added dropwise and the reaction was left for 30 minutes. 1 g of anthraquinone was added and the bath was removed. After 3 hours, 3 mL of iodomethane were added. After 18 hours, the solution was extracted with a saturated aqueous solution of ammonium chloride and dried with magnesium sulphate. The solvent was removed under vacuum and the residue was recrystallised twice from pentane to obtain 2.16 g of product as white flakes (yield: 54%).

### Example C - Synthesis of the precursor 4,8-bis((trimethylsilyl)ethynyl)-4,8-bis((trimethylsilyl)oxy)-4,8-dihydrobenzo[1,2-b:4,5-b']dithiophene:

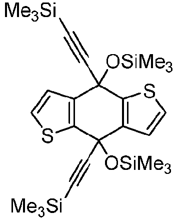

In a 50 mL round-bottom flask under argon, 3.2 mL of trimethylsilylacetylene were dissolved in 20 mL of dry THF and the solution was placed in an ice bath. 2.3 mL of methyllithium 1.6 M in diethyl ether were added dropwise and the reaction was left for 30 minutes. 330 mg of benzodithiophene-4,8-quinone were added and after 20 minutes the bath was removed. After 2 hours, 1 mL of chlorotrimethylsilane was added. After 2 hours, the solution was extracted with a saturated aqueous solution of ammonium chloride and dried with magnesium sulphate. The solvent was removed under vacuum and the residue was recrystallised twice from ethyl acetate to obtain 293 mg of product as small orange crystals (yield: 39%).

### Example D - Synthesis of the precursor ((2,6-dibromo-9,10-bis(phenylethynyl)-9,10-dihydroanthracene-9,10-diyl)bis(oxy))bis(trimethylsilane):

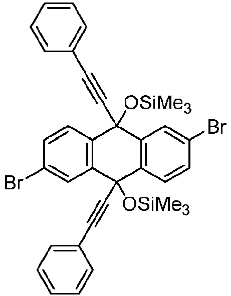

In a 100 mL round-bottom flask under argon, 0.2 mL of phenylacetylene were dissolved in 20 mL of dry THF and the solution was placed in an ice bath. 2.6 mL of tert-butyllithium 1.7 M in hexane were added dropwise and the reaction was left for 20 minutes. 650 mg of 2.6-dibromoanthraquinone were added and the bath was removed. After 4 hours, 1 mL of chlorotrimethylsilane was added. After 5 minutes, the solution was extracted with a saturated aqueous solution of ammonium chloride and dried with magnesium sulphate. The solvent was removed under vacuum and the residue was recrystallised twice from hexane to obtain 420 mg of product as white crystals (yield: 33%).

### Example E - Synthesis of the precursor ((2,6-dibromo-9,10-di(oct-1-yn-1-yl)-9,10-dihydroanthracene-9,10-diyl)bis(oxy))bis(trimethylsilane):

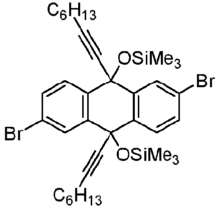

In a dry 100 mL round-bottom flask under nitrogen, 0.7 mL of 1-octyne were dissolved in 20 mL of dry THF and the solution was placed in an ice bath. 2.8 mL of methyllithium 1.6 M in diethyl ether were added dropwise and the reaction was left for 30 minutes. 650 mg of 2,6-dibromoanthraquinone were added and the bath was removed. After 4 hours, 1 mL of chlorotrimethylsilane was added. After 5 minutes, the solution was extracted with a saturated aqueous solution of ammonium chloride and dried with magnesium sulphate. The solvent was removed under vacuum and the residue was recrystallised twice from ethyl acetate to obtain 590 mg of product as white crystals (yield: 45%).

### Example F - Synthesis of the precursor ((9,10-bis((trimethylsilyl)ethynyl)-2,6-bis(trimethylstannyl)-9,10-dihydroanthracene-9,10-diyl)bis(oxy))bis(trimethylsilane):

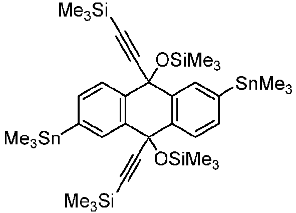

In a dry 50 mL Schlenk flask under nitrogen, 300 mg of ((2,6-dibromo-9,10-bis((trimethylsilyl)ethynyl)-9,10-dihydroanthracene-9,10-diyl)bis(oxy))bis(trimethylsilane) were dissolved in 15 mL of dry THF and the solution was placed in an ethanol-liquid nitrogen bath at -80 °C. 0.42 mL of n-butyllithium 2.5 M in hexane were added dropwise and the reaction was left for 2 hours before being brought back slowly to room temperature. The reaction was then brought back to -80 °C and 220 mg of trimethyltin chloride were added. After 3 hours the reaction was diluted with diethyl ether, extracted with water and dried with magnesium sulphate. The solvent was removed under vacuum and the residue was recrystallised twice from ethyl acetate to obtain 180 mg of product as white crystals (yield: 49%).

### Example G - Synthesis of the precursor ((2,6-di(thiophen-2-yl)-9,10-bis((triisopropylsilyl)ethynyl)-9,10-dihydroanthracene-9,10-diyl)bis(oxy))bis(trimethylsilane):

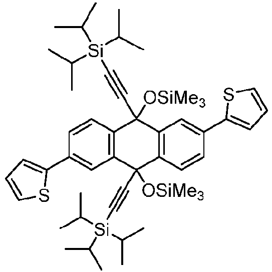

In a dry 50 mL Schlenk flask under nitrogen, 500 mg of ((2,6-dibromo-9,10-bis((triisopropylsilyl)ethynyl)-9,10-dihydroanthracene-9,10-diyl)bis(oxy))bis(trimethylsilane) were dissolved in 10 mL of dry toluene and the solution was degassed. 0.53 mL of 2-trimethylstannyl-thiophene and 16 mg of palladium XPhos Pd G2 were then added and the solution was heated to 110 °C for 6 hours. The reaction was then diluted with chloroform, extracted with water and dried with magnesium sulphate. The solvent was removed under vacuum and the residue was recrystallised twice from ethyl acetate to obtain 252 mg of product as a yellow powder (yield: 50%).

### Example H - Synthesis of the precursor 2,6-dibromo-9,10-bis((triisopropylsilyl)ethynyl)-9,10-dihydroanthracene-9,10-diol:

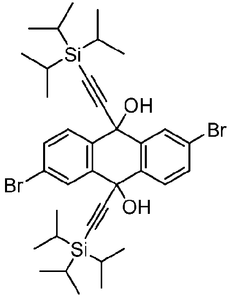

In a dry 100 mL round-bottom flask under nitrogen, 2.3 mL of (triisopropylsilyl)acetylene were dissolved in 20 mL of dry THF and the solution was placed in an ice bath. 3.9 mL of n-butyllithium 2.5 M in hexane were added dropwise and the reaction was left for 20 minutes. 1.5 g of 2,6-dibromoanthraquinone were added and the bath was removed. After 30 minutes, 5 mL of water were added. After 1 hour, the solution was extracted with a saturated aqueous solution of ammonium chloride and dried with magnesium sulphate. The solvent was removed under vacuum and the residue was recrystallised twice from acetone to obtain 2.19 g of product as a white powder (yield: 73%).

### Example I - Synthesis of the precursor (3,3-diphenyl-3-((trimethylsilyl)oxy)prop-1-yn-1-yl)trimethylsilane:

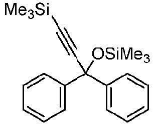

In a dry 100 mL round-bottom flask under nitrogen, 1.2 mL of trimethylsilylacetylene were dissolved in 20 mL of dry THF and the solution was placed in an ice bath. 5.1 mL of methyllithium 1.6 M in diethyl ether were added dropwise and the reaction was left for 15 minutes. 1 g of benzophenone was added and the bath was removed. After 2 hours, 1 mL of chlorotrimethylsilane was added. After 5 minutes, the solution was extracted with a saturated aqueous solution of ammonium chloride and dried with magnesium sulphate. The solvent was removed under vacuum and the residue was purified by chromatography on silica (petroleum ether: ethyl acetate 10:1, Rf=0.8) to obtain 1.58 g of product as a colourless oil (yield: 82%).

### Example J - Synthesis of the precursor ((2,6-dibromo-9,10-bis((methoxy)methoxy)-9,10-dihydroanthracene-9,10-diyl)bis(ethyn-2,1-diyl))bis(triisopropylsilane):

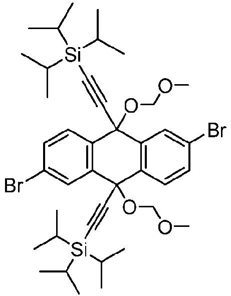

In a dry 100 mL round-bottom flask under nitrogen, 0.8 mL of (triisopropylsilyl)acetylene were dissolved in 20 mL of dry THF and the solution was placed in an ice bath. 2.1 mL of methyllithium 1.6 M in diethyl ether were added dropwise and the reaction was left for 15 minutes. 0.5 g of 2,6-dibromoanthraquinone were added and the bath was removed. After 30 minutes, 1.5 mL of bromomethyl methyl ether were added. After 10 minutes, the solution was extracted with a saturated aqueous solution of ammonium chloride and dried with magnesium sulphate. The solvent was removed under vacuum and the residue was recrystallised twice from acetone at - 20°C to obtain 162 mg of product as yellow-green needles (yield: 14%).

### Example K - Synthesis of the precursor 2,6-dibromo-9,10-diethynyl-9,10-dihydroanthracene-9,10-diol:

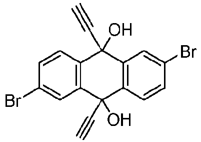

In a 100 mL round-bottom flask, 150 mg of ((2,6-dibromo-9,10-bis((trimethylsilyl)ethynyl)-9,10-dihydroanthracene-9,10-diyl)bis(oxy))bis(trimethylsilane) were suspended in 50 mL of methanol and 68 mg of potassium carbonate were added. After 18 hours, the reaction was poured into 150 mL of water containing 0.4 mL of hydrochloric acid 12 M. The white precipitate that formed was filtered, washed with abundant water and dried to obtain 88 mg of product as a white solid (yield: 99%).

### Example L - Synthesis of the precursor ((2,6-dichloro-9,10-dimethoxy-9,10-dihydroanthracene-9,10-diyl)bis(ethyn-2,1-diyl))bis(trimethylsilane):

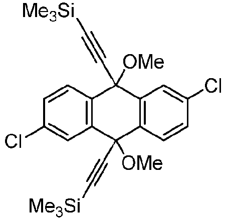

In a dry 100 mL round-bottom flask under nitrogen, 0.55 mL of trimethylsilylacetylene were dissolved in 20 mL of dry THF and the solution was placed in an ice bath. 2.3 mL of methyllithium 1.6 M in diethyl ether were added dropwise and the reaction was left for 5 minutes. 450 mg of 2,6-dichloroanthraquinone were added and the bath was removed. After 20 minutes, 3 mL of iodomethane were added and the reaction was heated to 50°C for 1 hour. After 18 hours, the solution was extracted with a saturated aqueous solution of ammonium chloride and dried with magnesium sulphate. The product was purified by column chromatography on silica (cyclohexane:diethyl ether 9:1, Rf=0.7) and recrystallised from methanol to obtain 274 mg of product as white needles (yield: 34%).

### Example L-bis - Synthesis of the precursor 10-methoxy-10-((trimethylsilyl)ethynyl)anthracen-9(10H)-one:

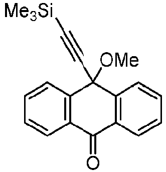

In a dry 100 mL round-bottom flask under nitrogen, 0.56 mL of trimethylsilylacetylene were dissolved in 20 mL of dry THF and the solution was placed in an ice bath. 2.34 mL of methyllithium 1.6 M in diethyl ether were added dropwise and the reaction was left for 10 minutes. 650 mg of anthraquinone were added and the bath was removed. After 1 hour, 1 mL of iodomethane was added. After 4 hours, the solution was extracted with a saturated aqueous solution of ammonium chloride and dried with magnesium sulphate. The product was purified by column chromatography on silica (hexane:ethyl acetate 9:1. Rf=0.9) and recrystallised from hexane to obtain 200 mg of product as white crystals (yield: 20%).

### Example M - Synthesis of the precursor 3,6-bis(phenylethynyl)-3,6-bis((trimethylsilyl)oxy)cyclohexa-1,4-diene:

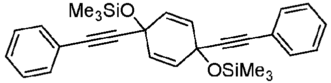

In a dry 100 mL round-bottom flask under nitrogen, 0.95 mL of phenylacetylene were dissolved in 20 mL of dry THF and the solution was placed in an ice bath. 5 mL of tert-butyllithium 1.7 M in pentane were added dropwise and the reaction was left for 10 minutes. 370 mg of benzoquinone were added and the bath was removed. After 45 minutes, 1 mL of chlorotrimethylsilane was added. After 5 minutes, the solution was extracted with a saturated aqueous solution of ammonium chloride and dried with magnesium sulphate. The solvent was removed under vacuum and the residue was recrystallised twice from pentane to obtain 657 mg of product as dirty white crystals (yield: 42%).

### Example N - Synthesis of the precursor 9,10-bis(bromoethynyl)-9,10-dimethoxy-9,10-dihydroanthracene:

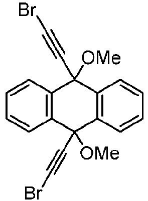

In a closed 25 mL round-bottom flask, 360 mg of 9,10-diethynyl-9,10-dimethoxy-9,10-dihydroanthracene were dissolved in 10 mL of acetone and the solution was degassed with argon. 200 mg of silver nitrate and 1.56 g of N-bromosuccinimide were added. The reaction was then poured onto ice and the precipitate was extracted with hot dichloromethane. The content of the organic phase was preabsorbed on silica and purified by column chromatography on silica (petroleum ether:ethyl acetate 2:1, followed by chloroform). 103 mg of product are obtained as white crystals (yield: 18%).

### Example O - Example of homo-polymerisation of ((2,6-dibromo-9,10-bis((butyldimethylsilyl)oxy)-9,10-dihydroanthracene-9,10-diyl)bis(ethylene-2,1-diyl))bis(triisopropylsilane):

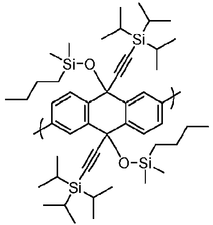

In a dry 50 mL Schlenk flask under argon, 200 mg of ((2,6-dibromo-9,10-bis((butyldimethylsilyl)oxy)-9,10-dihydroanthracene-9,10-diyl)bis(ethylene-2,1-diyl))bis(triisopropylsilane) (Example A), 53 mg of bis(pinacolato)diboron and 223 mg of tribasic potassium phosphate were dissolved in 8 mL of a 1:1 mixture of dry toluene and dry methylformamide. The solution was degassed and 16 mg of palladium(II) XPhos Pd G2 were added. The reaction was kept at 120 °C for 2 hours. 0.2 mL of 4-tertbutyl-1-bromobenzene were added and the reaction was left for a further hour. The solution was then poured into 200 mL of methanol and the precipitate was extracted with hot acetone and chloroform. The chloroform fraction was re-precipitated in methanol. The product was obtained as a yellow powder. Mw = 4126 Da, Mn = 3311

### Example P - Stille polymerisation of ((2,6-dibromo-9,10-bis((triisopropylsilyl)ethynyl)-9,10-dihydroanthracene-9,10-diyl)bis(oxy))bis(trimethylsilane) and 5,5'-bis(trimethylstannyl)-2,2'-bithiophene.

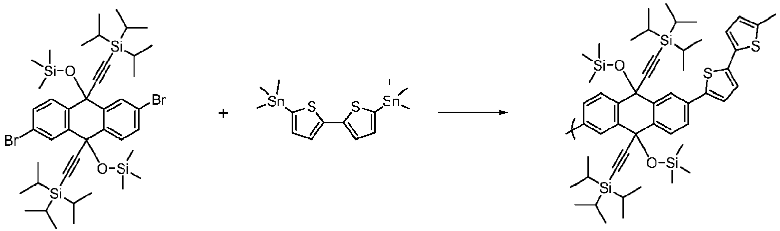

In a dry 50 mL Schlenk flask under argon, 300 mg of ((2,6-dibromo-9,10-bis((triisopropylsilyl)ethynyl)-9,10-dihydroanthracene-9,10-diyl)bis(oxy))bis(trimethylsilane) and 176 mg of 5,5'-bis(trimethylstannyl)-2,2'-bithiophene were dissolved in 22 mL of dry toluene and the solution was degassed. 17 mg of bis(dibenzylideneacetone)palladium(0) and 37 mg of tri(ortho-tolyl)phosphine were added and the reaction was left a 110 °C for 4 hours. 0.2 mL of 4-tertbutyl-1-bromobenzene were added and the reaction was left for a further hour. The solution was then poured into 200 mL of methanol and the precipitate was extracted with hot acetone and chloroform. The chloroform fraction was re-precipitated in methanol. The product was obtained as a green powder.

Using similar procedures it is possible obtain other copolymers:

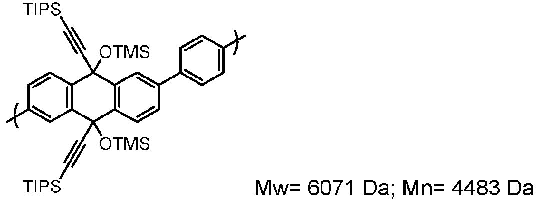

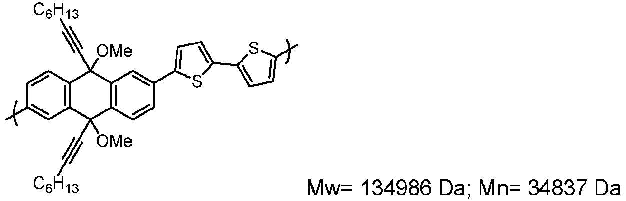

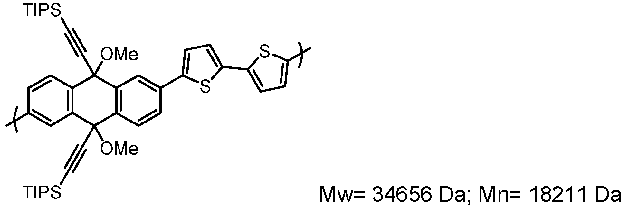

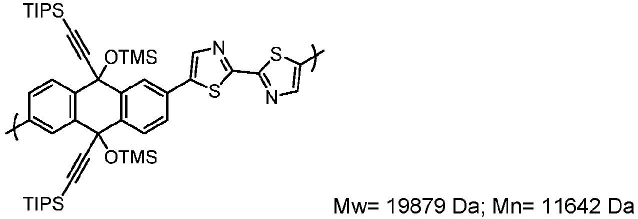

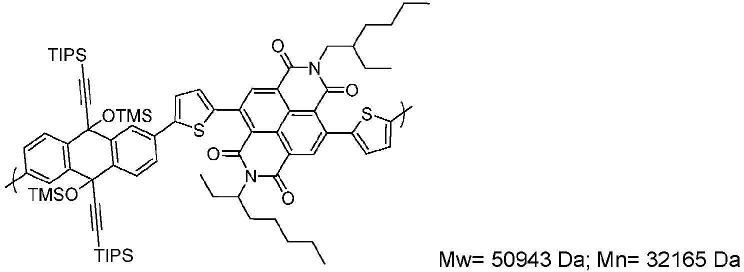

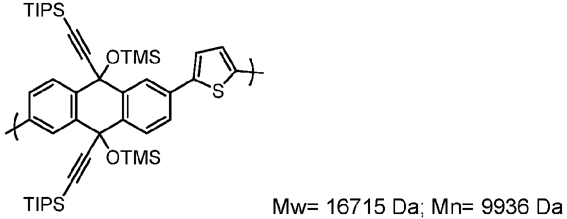

### Example Q - Formation of a high HOMO conjugated polymer from a polymeric precursor of example P.

50 mg of the polymer of example P were suspended in a solution obtained from 50 mg of tin dichloride, 0.1 mL of hydrochloric acid 12 M and 10 mL of methanol and heated to 50°C for 5 minutes. The solid was then thoroughly washed with water, methanol and acetone. The polymer obtained was not soluble and was characterised by IR spectroscopy (Figure 1).

### Example R - Formation of a low LUMO conjugated polymer from the polymeric precursor of example P.

50 mg of the polymer as per example P were dissolved in 15 mL of toluene. 100 mg of potassium hydroxide were added and the suspension was heated to 110°C for 1 hour. The precipitate was filtered and thoroughly washed with water and acetone. The polymer obtained was not soluble and was characterised by IR spectroscopy (Figure 1).

### Example S - Formation of a high HOMO conjugated polymer from a polymeric precursor of example P.

A film of the polymer of example P was obtained by spin coating (thickness 20 nm) and was placed on a plate at 100°C and covered with a few drops of a 10 mg/mL solution of 2-nitrobenzenesulfonylhydrazide. As soon as the solvent had evaporated, the sample was washed with methanol and acetone. The UV-visible absorption spectra and the cyclic voltammetry scan are shown respectively in Figure 2 A-B. As an electrochromic element, the polymer turned reversibly blue from red at +1 V against Ag/Ag+ (no other changes were observed up to -2.5 V).

### Example T - Formation of a low LUMO conjugated polymer from a polymeric precursor of example P.

A film of the polymer of example P obtained by spin coating (thickness 20 nm) was immersed in a solution obtained from 2.5 mL of tetrabutylammonium fluoride 1M in tetrahydrofuran and 2.5 mL of methyl isobutyl ketone and heated to 70°C for 5 minutes. It was then thoroughly washed with water, methanol and acetone. The UV-visible absorption spectra and the cyclic voltammetry scan are shown respectively in Figure 2 A-B. As an electrochromic element, the polymer turned reversibly green from brown at -1.5 V against Ag/Ag+ (no other changes were observed up to 1.5 V).

### Example U - Formation of regions of high HOMO and low LUMO polymeric materials on a polymeric precursor film.

A film of the polymer of example P obtained by spin coating on an indium-tin oxide sublayer (thickness 20 nm) was stamped in different regions using two different rubber stamps soaked in a solution of 0.1 M tin(II) chloride in 4:3 acetic acid:methanol and tetrabutylammonium fluoride 0.25 M in 1:4 tetrahydrofuran:methyl isobutyl ketone, respectively. In the two cases, stamping was carried out respectively at 70°C for 2 minutes and at 100°C for 30 seconds. After each stamping process, the film was thoroughly washed with abundant water and acetone.

### Example V (comparative) - Stille polymerisation of 2,6-dibromoanthraquinone or 2,6-dibromo-9,10-Bis[(triisopropylsilyl)ethynyl]anthracene and 5,5'-bis(trimethylstannyl)-2,2'-bithiophene

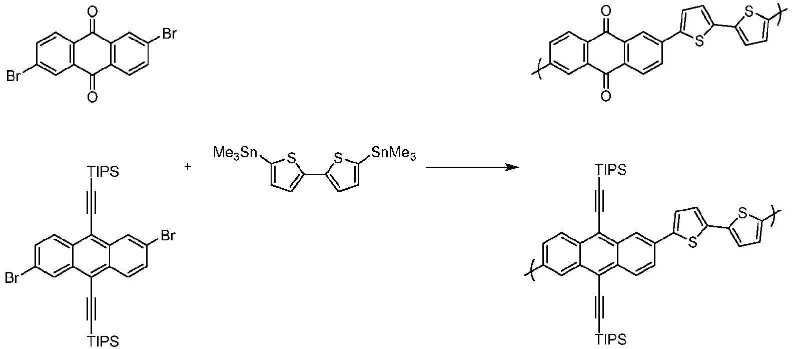

Using procedures similar to the one described in example P it is possible to prepare the high HOMO and low LUMO polymers described in examples Q-S and R-T, respectively.

In a dry 50 mL Schlenk flask under argon, 100 mg of 2,6-dibromoanthraquinone or 188 mg of 2,6-dibromo-9,10-bis(triisopropylsilylethynyl)-anthracene and 141 mg of 5,5'-bis(trimethylstannyl)-2,2'-bithiophene were dissolved in 25 mL of dry toluene and the solution was degassed. 16 mg of bis(dibenzylideneacetone)palladium(0) and 34 mg of tri(ortho-tolyl)phosphine were added and the reaction was left at 110 °C for 1 hour. Longer reaction times resulted in the production of abundant untreatable solid precipitate. 0.2 mL of 4-tertbutyl-1-bromobenzene were thus added and the reaction was left for a further hour. The solution was then poured into 200 mL of methanol and the precipitate was extracted with hot acetone and subsequently hot chloroform. The chloroform fraction was re-precipitated in methanol. The products were obtained as brown and red powders in the case of the polymer containing anthraquinone (low LUMO) and anthracene (high HOMO), respectively. The molecular weights measured by gel permeation chromatography (GPC) of solutions in chloroform were Mn 511 and Mw 734 in the case of the polymer containing anthraquinone units and Mn 1302 and Mw 1606 in the case of the polymer containing anthracene units, indicating that these materials are actually oligomers comprising between 2 and 4 units. These observations show that the use of a precursor is necessary to obtain high molecular weights.

### Example W - Example of formation of poly(9,10-diethylanthracene).

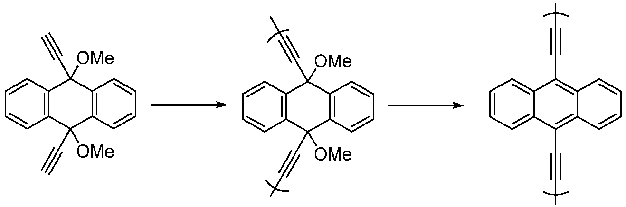

In a 20 mL vial, 215 mg of 9,10-diethynyl-9,10-dimethoxy-9,10-dihydroanthracene were dissolved in 5 mL of chloroform and the reaction was placed under vigorous stirring at 60°C. 14 mg of copper(I) iodide and 0.05 mL of tetramethylethylenediamine were added and the solution was left for 5 hours, maintaining a constant level of chloroform. The chloroform was then removed and the residue was extracted with water and acetone. The polymer was then suspended in a solution obtained from 50 mg of tin dichloride and 0.1 mL of hydrochloric acid 12 M in 10 mL of methanol.

### Example X - Synthesis of 4,8-bis((trimethylsilyl)ethynyl)benzo[1,2-b:4,5-b']dithiophene from 4,8-bis((trimethylsilyl)ethynyl)-4,8-bis((trimethylsilyl)oxy)-4,8-dihydrobenzo[1,2-b:4,5-b']dithiophene:

In a vial, 30 mg of a precursor (Example C) were suspended in 1 mL of isopropanol and 3 mL of a solution prepared with 100 mg of tin(II) chloride. 10 mL of methanol and 0.1 mL of hydrochloric acid 12 M. After 20 minutes the content of the vial was poured into 50 mL of water, the solid was filtered and thoroughly washed with water. The product was obtained as a green powder.

### Example Y - Synthesis of benzo[1,2-b:4,5-b']dithiophene-4,8-dione from 4,8-bis((trimethylsilyl)ethynyl)-4,8-bis((trimethylsil)oxy)-4,8-dihydrobenzo[1,2-b:4,5-b']dithiophene.

In a sealed vial, 27 mg of a starting precursor (Example C) were dissolved in 20 mL of tetrahydrofuran and 0.3 mL of tetrabutylammonium fluoride 1M in THF were added. The reaction was heated to 80°C for 30 minutes. The solvent was then removed under vacuum and the residual was washed with deionised water. The product was obtained as a brown powder.

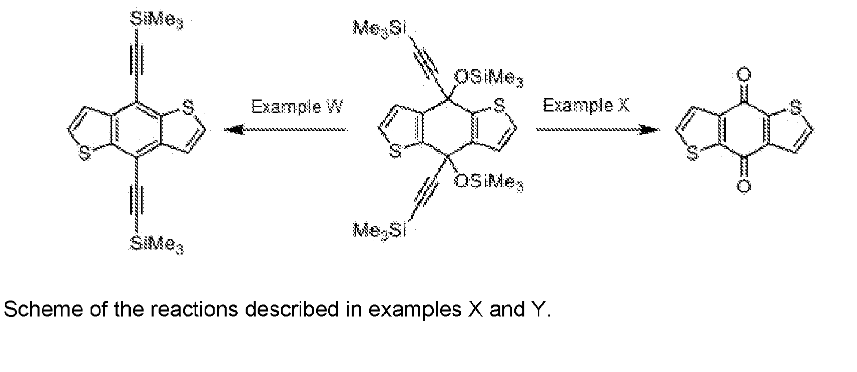

## Claims

1. Process for the production of an organic layer comprising a plurality of regions with different and complementary electronic properties, comprising the step of subjecting at least one precursor selected from the following compounds of formula (I), (II), (IIa), (III) and (IV), or combinations thereof, to a reduction/re-aromatisation reaction and a retro-Favorskii reaction: 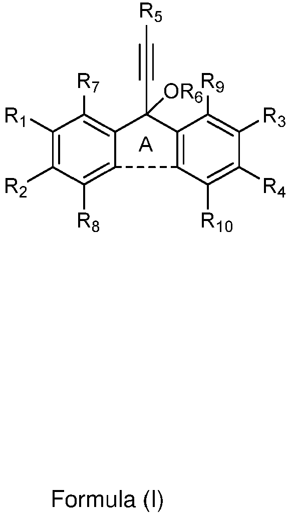 in which
R1, R2, R3, R4, R5, R7, R8, R9 and R10 are independently selected from: H, a halogen selected from Br, I, Cl, F, C1-C8 alkyl, vinyl, alkynyl, aryl, heteroaryl, benzyl, N, B, S, O, Sn, tri-alkyl-silane; R6 is selected from: H, C1-C8 alkyl, vinyl, alkynyl, aryl, benzyl, tri-alkyl-silane;
A indicates a 5-carbon atom ring or a 6-carbon atom ring optionally substituted with a propargyl alcohol group, in which the alcohol of the propargyl alcohol group is optionally substituted, or the A ring is not present; 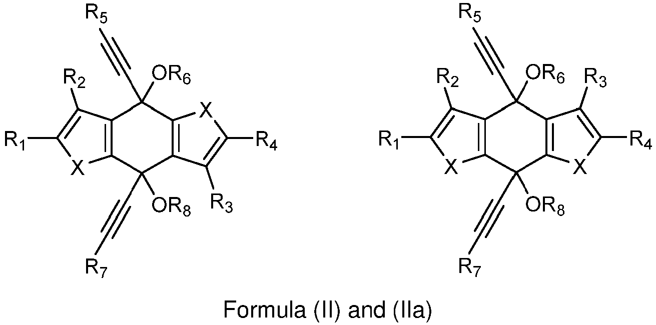
in which:
R1, R2, R3, R4, R5 and R7 are independently selected from: H, a halogen selected from Br, I, Cl, F, C1-C8 alkyl, vinyl, alkynyl, aryl, heteroaryl, benzyl, N, B, S, O, Sn, tri-alkyl-silane;
R6 and R8 are independently selected from: H, C1-C8 alkyl, vinyl, alkynyl, aryl, benzyl, tri-alkyl-silane;
X is selected from S, Se, N, O, methylene; 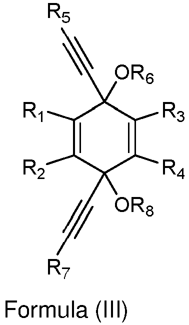
in which
R1, R2, R3, R4, R5 and R7 are independently selected from: H, a halogen selected from Br, I, Cl, F, C1-C8 alkyl, vinyl, alkynyl, aryl, heteroaryl, benzyl, N, B, S, O, Sn, tri-alkyl-silane;
R6 and R8 are independently selected from: H, C1-C8 alkyl, vinyl, alkynyl, aryl, benzyl, tri-alkyl-silane; 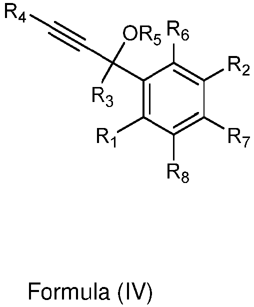
in which:
R1, R2, R4, R6, R7 and R8 are independently selected from: H, a halogen selected from Br, I, Cl, F, C1-C8 alkyl, vinyl, alkynyl, aryl, heteroaryl, benzyl, N, B, S, O, Sn, tri-alkyl-silane;
R3 is selected from: H, C1-C8 alkyl, vinyl, alkynyl, aryl, benzyl, N;
R5 is selected from: H, C1-C8 alkyl, vinyl, alkynyl, aryl, benzyl, tri-alkyl-silane,
wherein the reduction/re-aromatisation reaction is carried out with a reducing agent selected from: SnCl₂/H⁺, 2-nitrobenzenesulfonylhydrazide and PCl₃, while the retro-Favorskii reaction is carried out under strong basic conditions or by treating the precursor with a fluoride selected from tetrabutylammonium fluoride, KF and/or AgF.

2. Process according to claim 1, in which in the precursor of formula (I), A is a saturated 6-carbon atom ring substituted with a propargyl alcohol group according to formula (la): 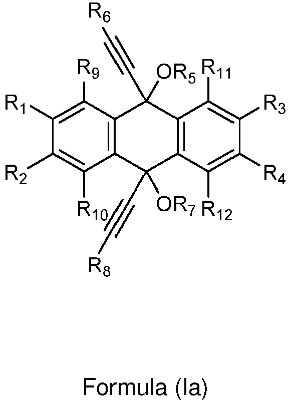 in which:
R1, R2, R3, R4, R6, R8, R9, R10, R11 and R12 are independently selected from: H, a halogen selected from Br, I, Cl, F, C1-C8 alkyl, vinyl, alkynyl, aryl, heteroaryl, benzyl, N, B, S, O, Sn, tri-alkyl-silane;
R5 and R7 are independently selected from: H, C1-C8 alkyl, vinyl, alkynyl, aryl, benzyl, tri-alkyl-silane.

3. Process according to claim 1 or 2, in which the reduction/re-aromatisation reaction leads to the formation of molecules, and thus regions, **characterised by** particularly high energy levels of the highest occupied molecular orbital (HOMO), while the retro-Favorskii reaction leads to the formation of molecules, and thus regions, **characterised by** particularly low energy levels of the lowest unoccupied molecular orbital (LUMO).

4. Process according to claim 3, wherein the molecules **characterised by** particularly high energy levels of the highest occupied molecular orbital (HOMO) comprise at least one aromatic ring substituted with at least one ethynyl group starting from the precursors of formula (la), (II), (IIa) and (III), while the precursor of formula (I) with A equal to a 5-carbon atom ring and the precursor of formula (IV) do not react under the reduction/re-aromatisation conditions.

5. Process according to claim 3 or 4, wherein the molecules **characterised by** particularly low energy levels of the lowest unoccupied molecular orbital (LUMO) comprise at least one carbonyl group for the precursors of formula (I) when A is a 5-carbon atom ring, or is not present, and for the precursor of formula (IV), or comprise at least one quinone starting from the precursors of formula (la), (II), (IIa) and (III).

6. Process according to any one of claims 1 to 5, wherein at least one precursor of formula (I), (la), (II), (IIa), (III) and (IV), or mixtures thereof, is subjected to a polymerisation reaction prior to being subjected to the reduction/re-aromatisation reaction and/or the retro-Favorskii reaction.

7. Process according to claim 6, wherein the polymerisation is either a homo-polymerisation or a co-polymerisation, with a co-monomer selected from: ethene, ethine, benzene, acenes, thiophene, 2,2'-bithiophene, bithiazole, fluorene, thieno[3,2-b]thiophene, dithieno[3,2-b:2',3'-d]thiophene, 1,4-dione-pyrrolo[3,4-c]pyrrole and 1,3,6,8(2H,7H)-tetraone-2,7-dialkylbenzo[Imn][3,8]phenanthroline.

8. Process according to claim 6 or 7, wherein the polymer obtained by polymerisation has a molecular weight between 2000 Da and 80000 Da, preferably between 20000 and 60000 Da.

9. Process according to any one of claims 1 to 5, wherein after the precursor of formula (I), (II), (IIa), (III) and (IV) has been subjected to the reduction/re-aromatisation reaction and/or the retro-Favorskii reaction, the obtained precursor is subjected to a polymerisation reaction selected from: a homo-polymerisation and/or a co-polymerisation with a co-monomer selected from: ethene, ethine, benzene, acenes, thiophene, 2,2'-bithiophene, bithiazole, fluorene, thieno[3,2-b]thiophene, dithieno[3,2-b:2',3'-d]thiophene, 1,4-dione-pyrrolo[3,4-c]pyrrole and 1,3,6,8(2H,7H)-tetraone-2,7-dialkylbenzo[lmn][3,8]phenanthroline.

10. An organic layer or organic film comprising a plurality of regions having different and complementary electronic properties, wherein the plurality of regions comprise one or more regions having electrical and electronic properties derived from a high HOMO and one or more regions having electrical and electronic properties derived from a low LUMO, wherein the one or more regions having electrical and electronic properties derived from a high HOMO comprise molecules and/or polymers obtained from the reduction/re-aromatisation reaction of a precursor of formula (la), (II), (IIa) or (III) as defined in claims 1 and 2, optionally polymerised before or after said reduction reaction, the one or more regions with electrical and electronic properties derived from a low LUMO comprise molecules and/or polymers obtained from the retro-Favorskii reaction of a precursor of formula (I), (la), (II), (IIa), (III) or (IV), as defined in claims 1 and 2, optionally polymerised before or after said retro-Favorskii reaction.

11. Use of the organic film according to claim 10, for the preparation of electronic devices, preferably transistors, more preferably organic field-effect transistors (OFET) and organic electrochemical transistors (OECT), or for the preparation of photovoltaic panels or in the formation of light-emitting diodes or in the preparation of electrochromic layers.

12. Use of a compound or polymer **characterised by** a high-energy HOMO obtained from the reduction/re-aromatisation reaction of at least one precursor of formula (la), (II), (IIa) and (III), as defined in claims 1 and 2, optionally polymerised before or after the reduction/re-aromatisation reaction, as an organic acceptor for polymeric photovoltaic cells or for the preparation of polymeric conductors following doping.

## Patentansprüche

1. Verfahren zur Herstellung einer organischen Schicht mit einer Vielzahl von Bereichen mit unterschiedlichen und komplementären elektronischen Eigenschaften, umfassend den Schritt zum Unterwerfen mindestens eines Vorläufers, der aus den folgenden Verbindungen der Formel (I), (II), (IIa), (III) und (IV) oder Kombinationen davon ausgewählt ist, einer Reduktions-/Rearomatisierungsreaktion und einer Retro-Favorskii-Reaktion: 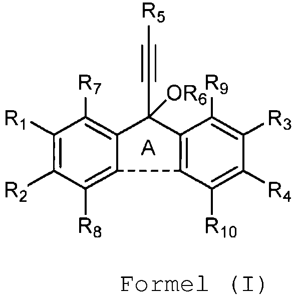 wobei
R1, R2, R3, R4, R5, R7, R8, R9 und R10 unabhängig ausgewählt sind, aus: H, einem Halogen, ausgewählt aus Br, I, Cl, F, C1-C8-Alkyl, Vinyl, Alkinyl, Aryl, Heteroaryl, Benzyl, N, B, S, O, Sn, Trialkylsilan;
R6 ausgewählt ist, aus: H, C1-C8-Alkyl, Vinyl, Alkinyl, Aryl, Benzyl, Trialkylsilan;
A einen 5-Kohlenstoffatomring oder einen 6-Kohlenstoffatomring, gegebenenfalls substituiert mit einer Propargylalkoholgruppe, angibt, wobei der Alkohol der Propargylalkoholgruppe gegebenenfalls substituiert ist oder der A-Ring nicht vorhanden ist; 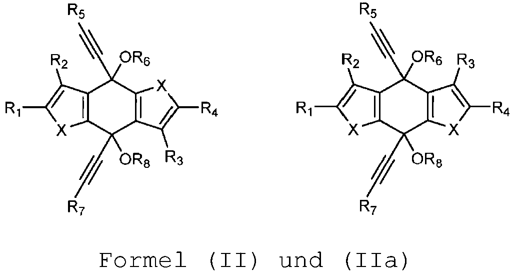
wobei:
R1, R2, R3, R4, R5 und R7 unabhängig ausgewählt sind, aus: H, einem Halogen, ausgewählt aus Br, I, Cl, F, C1-C8-Alkyl, Vinyl, Alkinyl, Aryl, Heteroaryl, Benzyl, N, B, S, O, Sn, Trialkylsilan;
R6 und R8 unabhängig ausgewählt sind, aus: H, C1-C8-Alkyl, Vinyl, Alkinyl, Aryl, Benzyl, Trialkylsilan;
X aus S, Se, N, O, Methylen ausgewählt ist; 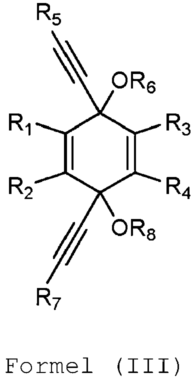
wobei
R1, R2, R3, R4, R5 und R7 unabhängig ausgewählt sind, aus: H, einem Halogen, ausgewählt aus Br, I, Cl, F, C1-C8-Alkyl, Vinyl, Alkinyl, Aryl, Heteroaryl, Benzyl, N, B, S, O, Sn, Trialkylsilan;
R6 und R8 unabhängig ausgewählt sind, aus: H, C1-C8-Alkyl, Vinyl, Alkinyl, Aryl, Benzyl, Trialkylsilan; 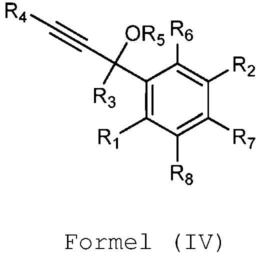
wobei:
R1, R2, R4, R6, R7 und R8 unabhängig ausgewählt sind, aus: H, einem Halogen, ausgewählt aus Br, I, Cl, F, C1-C8-Alkyl, Vinyl, Alkinyl, Aryl, Heteroaryl, Benzyl, N, B, S, O, Sn, Trialkylsilan;
R3 ausgewählt ist, aus: H, C1-C8-Alkyl, Vinyl, Alkinyl, Aryl, Benzyl, N;
R5 ausgewählt ist, aus: H, C1-C8-Alkyl, Vinyl, Alkinyl, Aryl, Benzyl, Trialkylsilan,
wobei die Reduktions-/Rearomatisierungsreaktion mit einem Reduktionsmittel durchgeführt wird, das ausgewählt ist aus: SnCl₂/H⁺, 2-Nitrobenzolsulfonylhydrazid und PCl₃, während die Retro-Favorskii-Reaktion unter stark basischen Bedingungen oder durch Behandlung des Vorläufers mit einem Fluorid, ausgewählt aus Tetrabutylammoniumfluorid, KF und/oder AgF, durchgeführt wird.

2. Verfahren nach Anspruch 1, wobei im Vorläufer der Formel (I) A ein gesättigter 6-Kohlenstoffatomring ist, der mit einer Propargylalkoholgruppe gemäß Formel (Ia) substituiert ist: 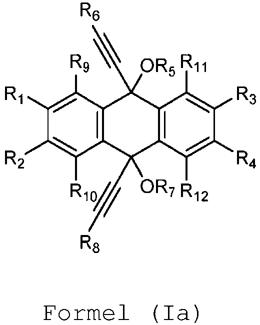 wobei:
R1, R2, R3, R4, R6, R8, R9, R10, R11 und R12 unabhängig ausgewählt sind, aus: H, einem Halogen, ausgewählt aus Br, I, Cl, F, C1-C8-Alkyl, Vinyl, Alkinyl, Aryl, Heteroaryl, Benzyl, N, B, S, O, Sn, Trialkylsilan;
R5 und R7 unabhängig ausgewählt sind, aus: H, C1-C8-Alkyl, Vinyl, Alkinyl, Aryl, Benzyl, Trialkylsilan.

3. Verfahren nach Anspruch 1 oder 2, wobei die Reduktions-/Rearomatisierungsreaktion zur Bildung von Molekülen und damit Bereichen führt, die durch besonders hohe Energieniveaus des höchsten besetzten Molekülorbitals (HOMO) gekennzeichnet sind, während die Retro-Favorskii-Reaktion zur Bildung von Molekülen und damit Bereichen führt, die durch besonders niedrige Energieniveaus des niedrigsten unbesetzten Molekülorbitals (LUMO) gekennzeichnet sind.

4. Verfahren nach Anspruch 3, wobei die Moleküle, die durch besonders hohe Energieniveaus des höchsten besetzten Molekülorbitals (HOMO) gekennzeichnet sind, mindestens einen aromatischen Ring umfassen, der mit mindestens einer Ethinylgruppe ausgehend von den Vorläufern der Formel (Ia), (II), (IIa) und (III) substituiert ist, während der Vorläufer der Formel (I) mit A gleich einem 5-Kohlenstoffatomring und der Vorläufer der Formel (IV) unter den Reduktions-/Rearomatisierungsbedingungen nicht reagieren.

5. Verfahren nach Anspruch 3 oder 4, wobei die Moleküle, die durch besonders niedrige Energieniveaus des niedrigsten unbesetzten Molekülorbitals (LUMO) gekennzeichnet sind, mindestens eine Carbonylgruppe für die Vorläufer der Formel (I), wenn A ein 5-Kohlenstoffatomring ist oder nicht vorhanden ist, und für den Vorläufer der Formel (IV) umfassen oder mindestens ein Chinon ausgehend von den Vorläufern der Formel (Ia), (II), (IIa) und (III) umfassen.

6. Verfahren nach einem der Ansprüche 1 bis 5, wobei mindestens ein Vorläufer der Formel (I), (Ia), (II), (IIa), (III) und (IV) oder Mischungen davon einer Polymerisationsreaktion unterzogen wird, bevor er der Reduktions-/Rearomatisierungsreaktion und/oder der Retro-Favorskii-Reaktion unterzogen wird.

7. Verfahren nach Anspruch 6, wobei die Polymerisation entweder eine Homopolymerisation oder eine Copolymerisation mit einem Comonomer ist, das ausgewählt ist aus: Ethen, Ethin, Benzol, Acenen, Thiophen, 2,2'-Bithiophen, Bithiazol, Fluoren, Thieno[3,2-b]thiophen, Dithieno[3,2-b:2',3'-d]thiophen, 1,4-Dion-pyrrolo[3,4-c]pyrrol und 1,3,6,8(2H,7H)-Tetraon-2,7-dialkylbenzo[lmn][3,8]phenanthrolin.

8. Verfahren nach Anspruch 6 oder 7, wobei das durch Polymerisation erhaltene Polymer ein Molekulargewicht zwischen 2000 Da und 80000 Da, vorzugsweise zwischen 20000 und 60000 Da, aufweist.

9. Verfahren nach einem der Ansprüche 1 bis 5, wobei, nachdem der Vorläufer der Formel (I), (II), (IIa), (III) und (IV) der Reduktions-/Rearomatisierungsreaktion und/oder der Retro-Favorskii-Reaktion unterzogen wurde, der erhaltene Vorläufer einer Polymerisationsreaktion unterzogen wird, die ausgewählt ist aus: einer Homopolymerisation und/oder einer Copolymerisation mit einem Comonomer, das ausgewählt ist aus: Ethen, Ethin, Benzol, Acenen, Thiophen, 2,2'-Bithiophen, Bithiazol, Fluoren, Thieno[3,2-b]thiophen, Dithieno[3,2-b:2',3'-d]thiophen, 1,4-Dion-pyrrolo[3,4-c]pyrrol und 1,3,6,8(2H,7H)-Tetraon-2,7-dialkylbenzo[lmn] [3,8]phenanthrolin.

10. Organische Schicht oder organischer Film mit einer Vielzahl von Bereichen mit unterschiedlichen und komplementären elektronischen Eigenschaften, wobei die Vielzahl von Bereichen einen oder mehrere Bereich(e) mit elektrischen und elektronischen Eigenschaften, die von einem hohen HOMO abgeleitet sind, und einen oder mehrere Bereich(e) mit elektrischen und elektronischen Eigenschaften, die von einem niedrigen LUMO abgeleitet sind, umfasst, wobei der eine oder die mehreren Bereich(e) mit elektrischen und elektronischen Eigenschaften, die von einem hohen HOMO abgeleitet sind, Moleküle und/oder Polymere umfassen, die aus der Reduktions-/Rearomatisierungsreaktion eines Vorläufers der Formel (Ia), (II), (IIa) oder (III), wie in den Ansprüchen 1 und 2 definiert, erhalten werden, optional polymerisiert vor oder nach der Reduktionsreaktion, wobei der eine oder die mehreren Bereich(e) mit elektrischen und elektronischen Eigenschaften, die von einem niedrigen LUMO abgeleitet sind, Moleküle und/oder Polymere umfassen, die aus der Retro-Favorskii-Reaktion eines Vorläufers der Formel (I), (Ia), (II), (IIa), (III) oder (IV), wie in den Ansprüchen 1 und 2 definiert, erhalten werden, optional polymerisiert vor oder nach der Retro-Favorskii-Reaktion.

11. Verwendung des organischen Films nach Anspruch 10 zur Vorbereitung von elektronischen Vorrichtungen, vorzugsweise Transistoren, bevorzugter organischen Feldeffekttransistoren (OFET) und organischen elektrochemischen Transistoren (OECT), oder zur Vorbereitung von Photovoltaikpaneelen oder zur Bildung von Leuchtdioden oder zur Vorbereitung von elektrochromen Schichten.

12. Verwendung einer Verbindung oder eines Polymers, **gekennzeichnet durch** ein hochenergetisches HOMO, erhalten aus der Reduktions-/Rearomatisierungsreaktion mindestens eines Vorläufers der Formel (Ia), (II), (IIa) und (III), wie in den Ansprüchen 1 und 2 definiert, gegebenenfalls polymerisiert vor oder nach der Reduktions-/Rearomatisierungsreaktion, als organischer Akzeptor für polymere photovoltaische Zellen oder zur Vorbereitung von polymeren Leitern nach der Dotierung.

## Revendications

1. Procédé de production d'une couche organique comprenant une pluralité de régions ayant des propriétés électroniques différentes et complémentaires, comprenant l'étape consistant à soumettre au moins un précurseur choisi parmi les composés suivants de formule (I), (II), (IIa), (III) et (IV), ou leurs combinaisons, à une réaction de réduction/ré-aromatisation et à une réaction de rétro-Favorskii : 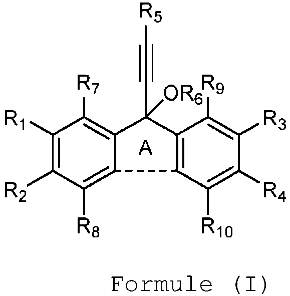 où
R1, R2, R3, R4, R5, R7, R8, R9 et R10 sont choisis indépendamment parmi : H, un halogène choisi parmi Br, I, Cl, F, alkyle en C1-C8, vinyle, alcynyle, aryle, hétéroaryle, benzyle, N, B, S, O, Sn, trialkylsilane ; R6 est choisi parmi : H, alkyle en C1-C8, vinyle, alcynyle, aryle, benzyle, trialkylsilane ;
A indique un cycle à 5 atomes de carbone ou un cycle à 6 atomes de carbone éventuellement substitué par un groupe alcool propargylique, dans lequel l'alcool du groupe alcool propargylique est éventuellement substitué, ou le cycle A n'est pas présent ; 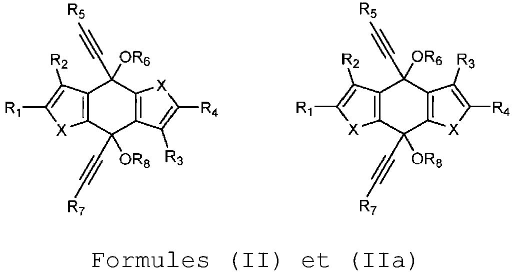
où :
R1, R2, R3, R4, R5 et R7 sont choisis indépendamment parmi : H, un halogène choisi parmi Br, I, Cl, F, alkyle en C1-C8, vinyle, alcynyle, aryle, hétéroaryle, benzyle, N, B, S, O, Sn, trialkylsilane ;
R6 et R8 sont choisis indépendamment parmi : H, alkyle en C1-C8, vinyle, alcynyle, aryle, benzyle, trialkylsilane ;
X est choisi parmi S, Se, N, O, méthylène ; 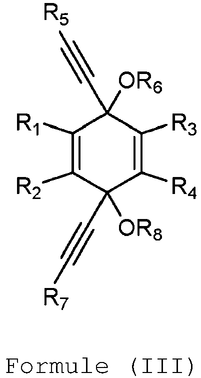
où
R1, R2, R3, R4, R5 et R7 sont choisis indépendamment parmi : H, un halogène choisi parmi Br, I, Cl, F, alkyle en C1-C8, vinyle, alcynyle, aryle, hétéroaryle, benzyle, N, B, S, O, Sn, trialkylsilane ;
R6 et R8 sont choisis indépendamment parmi : H, alkyle en C1-C8, vinyle, alcynyle, aryle, benzyle, trialkylsilane ; 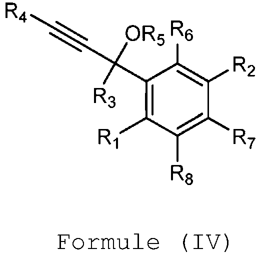
où :
R1, R2, R4, R6, R7 et R8 sont choisis indépendamment parmi : H, un halogène choisi parmi Br, I, Cl, F, alkyle en C1-C8, vinyle, alcynyle, aryle, hétéroaryle, benzyle, N, B, S, O, Sn, trialkylsilane ;
R3 est choisi parmi : H, alkyle en C1-C8, vinyle, alcynyle, aryle, benzyle, N ;
R5 est choisi parmi : H, alkyle en C1-C8, vinyle, alcynyle, aryle, benzyle, trialkylsilane,
dans lequel la réaction de réduction/ré-aromatisation est réalisée avec un agent réducteur choisi parmi : SnCl₂/H⁺, 2-nitrobenzènesulfonylhydrazide et PCl₃, tandis que la réaction de rétro-Favorskii est réalisée dans des conditions fortement basiques ou en traitant le précurseur avec un fluorure choisi parmi le fluorure de tétrabutylammonium, KF et/ou AgF.

2. Procédé selon la revendication 1, dans lequel, dans le précurseur de formule (I), A est un cycle saturé à 6 atomes de carbone substitué par un groupe alcool propargylique selon la formule (Ia) : 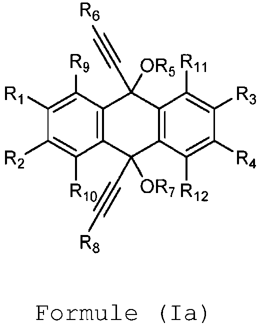 où :
R1, R2, R3, R4, R6, R8, R9, R10, R11 et R12 sont choisis indépendamment parmi : H, un halogène choisi parmi Br, I, Cl, F, alkyle en C1-C8, vinyle, alcynyle, aryle, hétéroaryle, benzyle, N, B, S, O, Sn, trialkylsilane ;
R5 et R7 sont choisis indépendamment parmi : H, alkyle en C1-C8, vinyle, alcynyle, aryle, benzyle, trialkylsilane.

3. Procédé selon la revendication 1 ou 2, dans lequel la réaction de réduction/ré-aromatisation conduit à la formation de molécules, et donc de régions, **caractérisées par** des niveaux d'énergie particulièrement élevés de l'orbitale moléculaire la plus haute occupée (HOMO), tandis que la réaction de rétro-Favorskii conduit à la formation de molécules, et donc de régions, **caractérisées par** des niveaux d'énergie particulièrement bas de l'orbitale moléculaire inoccupée la plus basse (LUMO) .

4. Procédé selon la revendication 3, dans lequel les molécules **caractérisées par** des niveaux d'énergie particulièrement élevés de l'orbitale moléculaire la plus haute occupée (HOMO) comprennent au moins un cycle aromatique substitué par au moins un groupe éthynyle à partir des précurseurs de formule (Ia), (II), (IIa) et (III), tandis que le précurseur de formule (I) avec A égal à un cycle à 5 atomes de carbone et le précurseur de formule (IV) ne réagissent pas dans les conditions de réduction/ré-aromatisation.

5. Procédé selon la revendication 3 ou 4, dans lequel les molécules **caractérisées par** des niveaux d'énergie particulièrement bas de l'orbital moléculaire inoccupée la plus basse (LUMO) comprennent au moins un groupe carbonyle pour les précurseurs de formule (I) lorsque A est un cycle à 5 atomes de carbone, ou n'est pas présent, et pour le précurseur de formule (IV), ou comprennent au moins une quinone à partir des précurseurs de formule (Ia), (II), (IIa) et (III).

6. Procédé selon l'une quelconque des revendications 1 à 5, dans lequel au moins un précurseur de formule (I), (Ia), (II), (IIa), (III) et (IV), ou des mélanges de ceux-ci, est soumis à une réaction de polymérisation avant d'être soumis à la réaction de réduction/ré-aromatisation et/ou à la réaction de rétro-Favorskii.

7. Procédé selon la revendication 6, dans lequel la polymérisation est soit une homopolymérisation, soit une copolymérisation, avec un comonomère choisi parmi : l'éthène, l'éthine, le benzène, les acènes, le thiophène, 2,2'-bithiophène, le bithiazole, le fluorène, thiéno[3,2-b]thiophène, dithiéno[3,2-b:2',3'-d]thiophène, 1,4-dione-pyrrolo[3,4-c]pyrrole et 1,3,6,8(2H,7H)-tétraone-2,7-dialkylbenzo[lmn][3,8]phénanthroline.

8. Procédé selon la revendication 6 ou 7, dans lequel le polymère obtenu par polymérisation a un poids moléculaire compris entre 2 000 Da et 80 000 Da, de préférence entre 20 000 et 60 000 Da.

9. Procédé selon l'une quelconque des revendications 1 à 5, dans lequel, après que le précurseur de formule (I), (II), (IIa), (III) et (IV) a été soumis à la réaction de réduction/ré-aromatisation et/ou à la réaction de rétro-Favorskii, le précurseur obtenu est soumis à une réaction de polymérisation choisie parmi : une homopolymérisation et/ou une copolymérisation avec un comonomère choisi parmi : l'éthène, l'éthine, le benzène, les acènes, le thiophène, 2,2'-bithiophène, le bithiazole, le fluorène, thiéno[3,2-b]thiophène, dithiéno[3,2-b:2',3'-d]thiophène, 1,4-dione-pyrrolo[3,4-c]pyrrole et 1,3,6,8(2H,7H)-tétraone-2,7-dialkylbenzo[lmn][3,8]phénanthroline.

10. Couche organique ou film organique comprenant une pluralité de régions ayant des propriétés électroniques différentes et complémentaires, dans laquelle/lequel la pluralité de régions comprend une ou plusieurs régions ayant des propriétés électriques et électroniques dérivées d'une HOMO haute et une ou plusieurs régions ayant des propriétés électriques et électroniques dérivées d'une LUMO basse, dans laquelle/lequel la ou les plusieurs régions ayant des propriétés électriques et électroniques dérivées d'une HOMO haute comprennent des molécules et/ou des polymères obtenu(e)s à partir de la réaction de réduction/ré-aromatisation d'un précurseur de formule (Ia), (II), (IIa) ou (III) tel que défini dans les revendications 1 et 2, éventuellement polymérisé(e)s avant ou après ladite réaction de réduction, la ou les plusieurs régions ayant des propriétés électriques et électroniques dérivées d'une LUMO basse comprennent des molécules et/ou des polymères obtenu(e)s à partir de la réaction de rétro-Favorskii d'un précurseur de formule (I), (Ia), (II), (IIa), (III) ou (IV), tel que défini dans les revendications 1 et 2, éventuellement polymérisé(e) s avant ou après ladite réaction de rétro-Favorskii.

11. Utilisation du film organique selon la revendication 10, pour la préparation de dispositifs électroniques, de préférence des transistors, plus préférablement des transistors à effet de champ organiques (OFET) et des transistors électrochimiques organiques (OECT), ou pour la préparation de panneaux photovoltaïques ou dans la formation de diodes électroluminescentes ou dans la préparation de couches électrochromiques.

12. Utilisation d'un composé ou d'un polymère **caractérisé par** une HOMO de haute énergie obtenu à partir de la réaction de réduction/ré-aromatisation d'au moins un précurseur de formule (Ia), (II), (IIa) et (III), tel que défini dans les revendications 1 et 2, éventuellement polymérisé avant ou après la réaction de réduction/ré-aromatisation, comme accepteur organique pour des cellules photovoltaïques polymères ou pour la préparation de conducteurs polymères après dopage.
